# EUROPÄISCHE PATENTSCHRIFT

(11) **EP 2 864 474 B1**
(45) Veröffentlichungstag und Bekanntmachung des Hinweises auf die Patenterteilung: **22.08.2018**
(21) Anmeldenummer: 13739622.2
(22) Anmeldetag: 21.06.2013
(51) Int. Cl.: C12N 5/077, A61F 2/28, A61F 2/30, A61K 35/22, A61K 35/28, A61K 35/34, A61K 35/407, A61L 27/38

(54) **VERFAHREN ZUR HERSTELLUNG VON FUNKTIONALEM FUSIONSGEWEBE**
METHOD FOR PRODUCING FUNCTIONAL FUSION TISSUE FROM HUMAN CHONDROCYTES
PROCÉDÉ DE FABRICATION D'UN TISSU DE FUSION FONCTIONNEL DE CHONDROCYTES HUMAINES

(30) Priorität: 21.06.2012 EP 12173056
(43) Veröffentlichungstag der Anmeldung: 29.04.2015
(73) Patentinhaber: Brandenburgische Technische Universität Cottbus-Senftenberg, 01968 Senftenberg (DE)
(72) Erfinder: ANDERER, Ursula, 01968 Senftenberg (DE); LEHMANN, Mario, 01968 Großkoschen (DE); MARTIN, Frank, 01968 Senftenberg (DE)
(74) Vertreter: Simandi, Claus
(86) Internationale Anmeldenummer: PCT/EP2013/062991
(87) Internationale Veröffentlichungsnummer: WO 2013/190088

(56) Entgegenhaltungen:
- US-B2- 7 887 843
- ANDERER U ET AL: "IN VITRO ENGINEERING OF HUMAN AUTOGENOUS CARTILAGE", JOURNAL OF BONE AND MINERAL RESEARCH, AMERICAN SOCIETY FOR BONE AND MINERAL RESEARCH, NEW YORK, NY, US, Bd. 17, Nr. 8, 1. August 2002 (2002-08-01) , Seiten 1420-1429, XP009026956, ISSN: 0884-0431, DOI: 10.1359/JBMR.2002.17.8.1420
- ANDERER U ET AL: "IN VITRO ENGINEERING OF HUMAN AUTOLOGOUS CARTILAGE", INTERNATIONAL JOURNAL OF ARTIFICIAL ORGANS, MILAN, IT, Bd. 25, Nr. 7, 1. Juli 2002 (2002-07-01), Seite 675, XP009026954, ISSN: 0391-3988
- FLEMING PAUL A ET AL: "Fusion of uniluminal vascular spheroids: a model for assembly of blood vessels.", DEVELOPMENTAL DYNAMICS : AN OFFICIAL PUBLICATION OF THE AMERICAN ASSOCIATION OF ANATOMISTS FEB 2010 LNKD- PUBMED:19918756, Bd. 239, Nr. 2, Februar 2010 (2010-02), Seiten 398-406, XP009164474, ISSN: 1097-0177
- VINATIER CLAIRE ET AL: "Cartilage tissue engineering: towards a biomaterial-assisted mesenchymal stem cell therapy.", CURRENT STEM CELL RESEARCH & THERAPY DEC 2009 LNKD- PUBMED:19804369, Bd. 4, Nr. 4, Dezember 2009 (2009-12), Seiten 318-329, XP000164461, ISSN: 1574-888X

## Beschreibung

Die vorliegende Erfindung betrifft ein universelles Verfahren zur Herstellung von funktionalem Fusionsgewebe. Gegenstand der Erfindung ist auch das durch dieses Verfahren erhältliche funktionale Fusionsgewebe sowie dessen Verwendung insbesondere als pharmazeutische Zubereitung, Arzneimittel, Transplantat, Implantat, Nahrungsmittel und Testsystem. Die Erfindung betrifft insbesondere die Herstellung von funktionalem Knorpel- und Knochengewebe aus Gelenkknorpel und Knochen.

Obwohl Gelenkknorpel eine bemerkenswerte Widerstandsfähigkeit zeigt, hat dieses Gewebe kein oder ein sehr geringes Vermögen zur Selbstreparatur und unbehandelte Läsionen können Osteoarthritis zur Folge haben. Dieses geringe Potential zur spontanen Regeneration führte zur Entwicklung von Zelltherapien, wie der autologen Chondrozytentransplantation (ACT), im Bestreben, eine funktionelle und schmerzlose Reparatur von Gelenkknorpeldefekten bereit zu stellen. Derartige Techniken können jedoch nicht die Knorpelregeneration garantieren und es gibt bisher keine ausreichenden Langzeitstudien. Demzufolge besteht hoher Bedarf an Methoden zur Knorpelregeneration, beispielsweise bei jungen aktiven Patienten mit traumatischen Läsionen oder sogar mit Symptomen einer Knorpeldegeneration.

Zahlreiche Studien wurden mit Chondrozyten durchgeführt, die aus Knorpel von Rindern, Kaninchen oder Schafen isoliert wurden. Die erhaltenen Daten und derartige tierbasierte Konzepte sind jedoch nicht auf die menschliche Situation übertragbar. Detaillierte biochemische und molekulare Studien mit humanen Chondrozyten wurden von einer Reihe von Faktoren behindert, wie der mangelnden Verfügbarkeit von Humangewebe in Verbindung mit der sehr geringen Anzahl von Zellen, die in einer Biopsie verfügbar sind, der begrenzten Prolieferationskapazität und der hohen phänotypischen Instabilität kultivierter Chondrozyten.

Ein Chondrozyt (Knorpelzelle), ist eine aus Chondroblasten hervorgehende und im Knorpelgewebe ansässige Zelle. Zusammen mit den Interzellularsubstanzen (extrazelluläre Matrix (EMZ)), bilden die Chondrozyten die Hauptbestandteile des Knorpels.

Es ist bereits bekannt, durch Mimikry bestimmter Prozesse der Embryonalentwicklung, z.B. menschliche Knorpelzellen dreidimensional auf einer Agaroseunterlage zu kultivieren, so dass Zellaggregate entstehen, die in ihrer Differenzierungsfähigkeit den Monolayerzellen überlegen sind und z.B. knorpelähnliche Eigenschaften aufweisen. Diese Eigenschaften, die möglichst genau das native Gelenkknorpelgewebe widerspiegeln, sind gekennzeichnet durch die Expression von Kollagen II (Hauptstrukturprotein der extrazellulären Matrix des hyalinen Knorpels), von Proteoglykanen, z.B. Aggrecan und dem intrazellulären chondrozytenspezifischen Protein S100. Darüber hinaus ist es wünschenswert dass die Expression von Kollagen I, welches während der Zellvermehrungsphase in der Monolayerkultur zwangsläufig hochreguliert wird, in den Zellaggregaten wieder reduziert wird da dieses Protein im nativen Gelenkknorpel praktisch nicht vorkommt. Die so generierten Zellaggregate (Sphäroide) werden seit 2002 beispielsweise von der Firma co.don angeboten um damit vor allem kleinere verletzungsbedingte Knorpeldefekte bei jüngeren Patienten zu therapieren. Dazu wird dem Patienten körpereigenes Gelenkknorpelgewebe entnommen und die daraus isolierten Zellen nach Vermehrung und 3D Kultur als Sphäroide (Chondrospheres®) transplantiert. Jedoch sind die so hergestellten Zellaggregate in ihrem Differenzierungsstatus begrenzt und zeigen häufig nur eine relativ schwache lokale Expression des wichtigen Kollagens II sowie nur marginale Synthese von Proteoglykanen bei jedoch relativ starker Expression des unerwünschten Kollagen I. Um die Differenzierung dieser Zellaggregate weiter zu verbessern, besteht die Möglichkeit das Kulturmedium mit bestimmten bioaktiven Substanzen anzureichern. Dazu zählen vorranging die vielfach in der Literatur beschriebenen Wachstumsfaktoren TGF-β1 - 3 sowie L-Ascorbinsäure (Vitamin C) als Kofaktor für die Kollagensynthese. Damit kann erreicht werden in den Zellaggregaten, die in Gegenwart dieser Faktoren kultiviert werden, vor allem die Proteoglykan- und Kollagen II Synthese zu verstärken. Allerdings reicht diese biochemische Stimulation zum einen oftmals nicht aus, um die Differenzierung der Knorpelzellen im 3D Zellaggregat soweit zu induzieren, dass knorpeltypische Konstrukte entstehen und zum anderen ist der Einsatz von Wachstumsfaktoren wie TGF-β insbesondere für eine klinische Anwendung am Menschen umstritten. Proteine der TGF-β- Familie regulieren eine Vielzahl zellulärer Prozesse wie z.B. Proliferation, Differenzierung, Wachstum, Migration, etc., können aber auch pathologisch an der Tumorentstehung beteiligt sein bzw. das Wachstum bereits bestehender Tumoren fördern sowie deren Metastasierung begünstigen.

Da ohne eine gegebene Gerüststruktur die Selbstorganisation eines Gewebes meist nicht gegeben ist, ist die Verwendung von Gerüsten (Scaffolds) oftmals entscheidend, wie beispielsweise beim Tissue Engeneering (TE) von Blutgefäßen. Dabei ist die Auswahl eines passenden Gerüsts in der Praxis schwierig und oft nicht möglich.

Vinatier et al. (Current Stem Cell Research & Therapy (2009), LNK-PUBMED: 19804369, Bd. 4, Nr. 4, S. 318-329) gibt einen Überblick über die Faktoren und Gerüstmaterialen, die beim Tissue Engineering von Knorpel verwendet werden.

Fleming et al. (Developmental Dynamics: An official publication of the American Association of Anatomists (2010) LNK-PUBMED: 19918756, Bd. 239, Nr. 2, S. 398-406) offenbart die Herstellung künstlicher Blutgefäße, wobei zwei vaskuläre Sphäroide, die jeweils ein zentrales Lumen haben, zu einem Sphäroid mit größerem Durchmesser und größerem zentralen Lumen fusioniert werden.

Anderer et. al (International Journal of Artificial Organs (2002), Milan, IT, Bd. 25, Nr. 7, S. 675) beschreibt ein Verfahren zur in vitro Herstellung von autologem Fusionsgewebe aus Knorpel, ohne jedoch die Fusion von Sphäroiden zu erwähnen. Das dadurch hergestellte Gewebe unterscheidet sich von nativem Gewebe deutlich im Hinblick auf die Expression extrazellulärer Matrixproteine, die für funktionales Knorpelgewebe notwendig sind.

Die Kultivierung von humanen Zellen h in Form von dreidimensionalen Zellaggregaten, z.B. in Form von sogenannten Sphäroiden, ist bereits zur klinischen Anwendung am Menschen, z.B. als autologes Knorpeltransplantat, zugelassen (DE 100 13 223).DE 100 13 223 betrifft ein Verfahren zur in vitro Herstellung von dreidimensionalem Knorpel- und Knochengewebe aus Knochen-, Knorpel- oder mesenchymalen Stammzellen. Dabei werden die Zellen zunächst in einer Monolayerkultur und anschließend in Suspension so lange kultiviert, bis ein Zellaggregat entsteht, das zumindest 40 Vol % extrazelluläre Matrix beinhaltet, in welches differenzierte Zellen eingebettet sind. In dem Verfahren werden Zellaggregate dadurch hergestellt, dass Zellen für mindestens 1-2 Wochen in Agarose beschichteten Zellkulturgefäßen kultiviert werden. DE 100 13 223 offenbart auch, dass je nach gewünschter Gewebegröße mindestens zwei der entstandenen Zellaggregate fusioniert werden, ohne jedoch konkrete Bedingungen für die Fusion zu nennen. Die nach diesem Verfahren erzeugten Gewebe unterscheiden sich hinsichtlich ihrer Funktionalität und Expressionsmuster, insbesondere im Hinblick auf die Expression von Kollagen Typ II, jedoch deutlich von den entsprechenden nativen Geweben.

US 7 887 843 B2 offenbart ein Verfahren zur in vitro Erzeugung von dreidimensionalem Knorpel- und Knochengewebe, wobei Sphäroide aus Knochen-, Knorpel- oder mesenchymalen Stammzellen dadurch hergestellt werden, dass 1 x 10⁵ Zellen für mindestens zwei Wochen kultiviert werden. Die gemäß US 7 887 843 B2 hergestellten Sphäroide haben nach einer Woche einen Durchmesser von 500 - 700 µm. Aus dieser Zellkultur erhält man, ohne den Verfahrensschritt der Fusion von Sphäroiden, nach 3 Monaten Fusionsgewebe mit einem Gehalt an extrazellulärer Matrix (EMZ) von 90 %. US 7 887 843 B2 nennt die Möglichkeit, in einem zweiten Schritt zwei oder mehr Sphäroide zu fusionieren, um größere Gewebestücke zu erzeugen, ohne hierfür jedoch konkrete Bedingungen anzugeben. Anderer et al. (Journal of Bone and Mineral Research (2002), New York, NY, US, Bd. 17, Nr. 8, S. 1420-1429) offenbart ebenfalls ein Verfahren zur in vitro Erzeugung von dreidimensionalem Knorpelgewebe. Nach diesem Verfahren werden 1 x 10⁵ oder 2 x 10⁵ Chrondrozyten für 5 Tage, 2 Wochen, 1, 2 und 3 Monate kultiviert, um Spähroide herzustellen. Die Nährstoffversorgung innerhalb der Sphäroide wird dadurch sichergestellt, dass nur Sphäroide mit einer Größe von weniger als 800 µm verwendet werden und wobei in einem zweiten Schritt 2 - 10 Sphäroide koalesziert werden können. Die hierfür verwendeten Sphäroide haben einen Durchmesser von 350 - 500 µm. Anderer et al. zeigt auch die Fusion von drei 16 Tage alten Sphäroiden.

Die im Stand der Technik beschriebenen in vitro hergestellten Knorpelgewebe zeigen keine Expressionsmuster essentieller Matrixproteine wie Kollagen Typ II, die denen der nativen Geweben ähnlich sind. Vielmehr weicht die Zusammensetzung der extrazellulären Matrix (EMZ), die in vitro von den Chrondrozyten erzeugt wird, deutlich von der nativer Knorpelgewebe ab. Die Zusammensetzung der EMZ ist jedoch entscheidend für die biologische Funktionalität des Knorpels, beispielsweise die mechanische Belastbarkeit. Es besteht deshalb ein Bedarf an neuen Techniken zur Erzeugung von funktionalem Fusionsgewebe, insbesondere von funktionalem Knorpelgewebe.

Die Erfindung stellt Techniken und Mittel zur Verfügung, mit denen funktionale Gewebe erzeugt werden können. Sie betrifft funktionale Fusionsgewebe, die nach einem neuen Verfahren hergestellt werden und die im Bezug auf ihre biologische Funktionalität denen des nativen Gewebes entsprechen bzw. weitgehend entsprechen. Beispielsweise findet sich eine sehr hohe Übereinstimmung im Hinblick auf die Expression von Kollagen Typ II und spezifischen Proteoglykanen, wenn nach dem erfindungsgemäßen Verfahren erzeugtes in vitro Knorpelgewebe mit nativem Knorpelgewebe verglichen wird. Das erfindungsgemäße Verfahren ist dadurch gekennzeichnet, dass die in vitro Kultivierung in einer besonderen dreidimensionalen Umgebung (3D-Umgebung) durchgeführt wird. Die erfindungsgemäße dreidimensionale Umgebung wird durch die Größe und Anzahl der verwendeten Sphäroide erreicht. Diese dreidimensionale Umgebung bewirkt, dass die Sphäroide spontan und selbstständig fusionieren. Mit dem erfindungsgemäßen Verfahren können, im Vergleich zu den im Stand der Technik beschriebenen Verfahren, größere Fusionsgewebe in einer kürzeren Zeit hergestellt werden, was sich überraschenderweise als besonders vorteilhaft auf die biologische Funktionalität des erzeugten Gewebes auswirkt.

Gegenstand der Erfindung ist ein Verfahren zur Herstellung von funktionalem Fusionsgewebe, dadurch gekennzeichnet, dass Sphäroide hergestellt und Sphäroide mit einem Duchmesser von mindestens 800 µm, vorzugsweise mit einem Durchmesser von 800 - 1400 µm, ausgewählt werden und wobei mindestens 5 Sphäroide mit einem Durchmesser von mindestens 800 µm, vorzugsweise mit einem Durchmesser von 800 - 1400 µm, fusioniert werden.

In dem erfindungsgemäßen Verfahren werden vorzugsweise Zellen aus Gewebe humanen oder tierischen Ursprungs verwendet, die aus dem Gewebe isoliert und aus denen die Sphäroide hergestellt werden. In einer Ausführungsform der Erfindung werden Späroide dadurch hergestellt, dass Zellen aus ihrer normalen Umgebung isoliert und kultiviert, d.h. vermehrt werden.

Durch die Isolierung der Zellen aus dem Gewebe dedifferenzieren die Zellen in der Regel ganz oder teilweise. Das erfindungsgemäße Verfahren eignet sich zur Herstellung von funktionalem Fusionsgewebe aus dedifferenzierten Zellen. Eine Ausführungsform des Verfahrens betrifft deshalb die Verwendung von dedifferenzierten Zellen zur Herstellung der Sphäroide. Alternativ können aber auch nicht oder nur teilweise dedifferenzierte Zellen zur Herstellung der Sphäroide verwendet werden.

Gegenstand der Erfindung ist ein Verfahren zur Herstellung von funktionalem Fusionsgewebe dadurch gekennzeichnet, dass
a) Zellen aus Gewebe humanen oder tierischen Ursprungs isoliert werden,
b) die isolierten Zellen in eine andere Umgebung gebracht und vermehrt werden,
c) aus den vermehrten Zellen Sphäroide hergestellt werden,
d) fünf oder mehr Sphäroide mit einem Durchmesser von 800 µm, vorzugsweise von 800 - 1400 µm, fusioniert werden.

Gegenstand der Erfindung ist auch ein Verfahren zur Herstellung von funktionalem Fusionsgewebe dadurch gekennzeichnet, dass
a) Zellen aus Gewebe humanen oder tierischen Ursprungs isoliert werden,
b) aus den isolierten Zellen dedifferenzierte Zellen hergestellt werden,
c) aus den dedifferenzierten Zellen Sphäroide hergestellt werden,
d) fünf oder mehr Sphäroide mit einem Durchmesser von 800 µm, vorzugsweise von 800 - 1400 µm, fusioniert werden.

In einer Ausführungsform werden Sphäroide hergestellt, die unterschiedliche Durchmesser haben und wobei in einem zweiten Schritt von den hergestellten Sphäroiden diejenigen ausgewählt werden, die einen Durchmesser von mindestens 800 µm, vorzugsweise von 800 - 1400 µm, haben.

In einer anderen Ausführungsform werden direkt Sphäroide hergestellt, die einen Durchmesser von mindestens 800 µm, vorzugsweise von 800 - 1400 µm, haben. Sphäroide dieser Größe können beispielsweise dadurch erhalten werden, dass 3 x 10⁵ bis 5 x 10⁵ Zellen pro Well einer Mikrotiterplatte (96-Well-Platte), vorzugsweise 4 x 10⁵ Zellen pro Well, besonders bevorzugt 3 x 10⁵ Zellen pro Well ausgesät werden. Diese Zellen können 1 bis 3 Tage, vorzugsweise 36 bis 60 Stunden, besonders bevorzugt 2 Tage kultiviert werden, um Sphäroide mit einer Größe von mindestens 800 µm, vorzugsweise von 800 - 1400 µm Durchmesser, herzustellen.

Für das Verfahren sind Sphäroide besonders geeignet, die einen Durchmesser von 800 µm, 900 µm, 1000 µm, 1100 µm, 1200 µm, 1300 µm, 1400 µm oder mehr haben.

Gegenstand der Erfindung ist ein Verfahren mit dem ohne weitere Hilfen wie Gerüste funktionales Fusionsgewebe, auch "Fusionskultursystem", "Fusionskultur" und "funktionales Gewebe" genannt, hergestellt werden kann. Das mit diesem Verfahren hergestellte funktionale Fusionsgewebe besteht dabei aus Fusionen, d.h. mehreren miteinander fusionierten Sphäroiden. Das mit Hilfe des erfindungsgemäßen Verfahrens hergestellte funktionale Fusionsgewebe liegt vorzugsweise in Form von Mikrogewebe vor.

"Funktionales Fusionsgewebe" bedeutet, dass das Gewebe weitgehend dem nativen Gewebe entspricht oder mit nativem Gewebe identisch ist. Im Sinne dieser Erfindung entspricht das funktionale Fusionsgewebe beispielsweise im Bezug auf die Expression bzw. Expressionsmuster der extrazellulären Matrix weitgehend dem des nativen Gewebes , beispielsweise im Bezug auf die Expression bzw. Expressionsmuster struktureller Makromoleküle wie Kollagen, insbesondere Kollagen Typ II und/oder Kollagen Typ I und/oder S100 Protein und/oder der gewebespezifischen Proteoglykane, z.B. knorpelspezifische Proteoglykane (bei funktionalem Knorpelgewebe, beispielsweise Gelenkknorpelgewebe). Der Nachweis der Expression bzw. Expressionsmuster kann z.B. histologisch oder immunhistologisch erfolgen. Bei funktionalem Fusionsgewebe anderer Gewebetypen kann der Nachweis beispielsweise über spezifische Oberflächenproteine oder Komponenten der extrazellulären Matrix erfolgen, deren Expressionsmuster in dem funktionalen Fusionsgewebe dem der betreffenden nativen Gewebe entspricht bzw. weitgehend entspricht.

Im Rahmen der vorliegenden Erfindung werden für die Herstellung von funktionalem Fusionsgewebe vorzugsweise Zellen verwendet, die in engem funktionellen Zusammenhang stehen und ein Gewebe bilden können oder Teil eines Gewebes sind, beispielsweise Fibroblasten, Hepatozyten, Nervenzellen, Osteoblasten, Osteoklasten, Keratinozyten. Das Gewebe, aus dem die Zellen isoliert werden, ist beispielsweise ausgewählt aus muskoskelletalem Gewebe, Skelettgewebe, Knorpel, Knochen, Meniskus, Epithelgewebe, Bindegewebe, Stützgewebe, Muskelgewebe, glatter Muskulatur, Herzmuskulatur, Nervengewebe, Funktionsgewebe (Parenchym), Zwischengewebe (Interstitium), Organgewebe, beispielsweise von Leber, Niere, Nebennierenrinde, Magen, Bauchspeicheldrüse, Herz, Lunge, Haut, Augenhornhaut, subkutanem Gewebe, Verdauungstrakt, Knochenmark, Gehirn, Schilddrüse, Milz, Gelenk, Sehne. Das nach dem erfindungsgemäßen Verfahren hergestellte funktionale Fusionsgewebe ähnelt dabei dem nativen Gewebe hinsichtlich der Zusammensetzung und Expression einer oder mehrerer Komponenten, vorzugsweise von mindestens zwei Komponenten. Funktionales Fusionsgewebe aus Chondrozyten entspricht dem nativen Knorpelgewebe beispielsweise hinsichtlich der Expression von Kollagen Typ I und Typ II, S100 Protein und / oder gewebespezifischen Proteoglykanen.

Natives Gelenkknorpelgewebe hat eine anteilsmäßige Zusammensetzung der extrazellulären Matrix von etwa 60 bis 80% Wasser im Bezug auf das Feuchtgewichts des Knorpelgewebes. Der hohe Wasseranteil ist wichtig für die mechanische Belastbarkeit des Knorpelgewebes und zusammen mit den Proteoglykanen für die "Schwammwirkung". Neben Wasser enthält das native Knorpelgelenk die strukturellen Makromoleküle der Matrix, wie Kollagene, Proteoglykane und nicht-Kollagen Proteine. Dabei machen die strukturellen Makromoleküle etwa 20 bis 40% des Feuchtgewichts des Gelenkknorpelgewebes aus.

Das funktionale Fusionsgewebe, das nach dem erfindungsgemäßen Verfahren aus humanen Chrondrozyten hergestellt wird, hat beispielsweise einen Wasseranteil von 50 bis 90 %, vorzugsweise 55 bis 85 % oder 60 bis 80 %, besonders bevorzugt 65 bis 75 % oder 70 % bezogen auf das Feuchtgewicht des funktionalen Fusionsgewebes. Das funktionale Fusionsgewebe hat beispielsweise 10 bis 50 % strukturelle Makromoleküle der Matrix, vorzugsweise 15 bis 45 % oder 20 bis 40 %, besonders bevorzugt 25 bis 35 % oder 30 % bezogen auf das Feuchtgewicht des funktionalen Gewebes. Dabei addieren sich die jeweiligen Wasseranteile, strukturellen Makromoleküle und gegebenenfalls weiteren Bestandteile zu 100 %.

Bei nativem Gelenkknorpelgewebe beträgt der Kollagenanteil 60%, der Proteoglykananteil 25 bis 35% und der nicht-Kollagenprotein- und Glycoproteinanteil 15 bis 20% im Bezug auf das Knorpeltrockengewicht. Das Hauptkollagen ist dabei Kollagen Typ II. Kollagen Typ II macht etwa 90 bis 95% des Gesamtkollagengehalts des nativen Gelenkknorpelgewebes aus.

Funktionales Fusionsgewebe, das nach dem erfindungsgemäßen Verfahren aus humanen Chrondrozyten hergestellt wird, enthält beispielsweise 50 bis 70 %, vorzugsweise 55 bis 65 %, besonders bevorzugt 60 % Kollagen im Bezug auf das Trockengewicht des funktionalen Fusionsgewebes.

Funktionales Fusionsgewebe, das nach dem erfindungsgemäßen Verfahren aus humanen Chrondrozyten hergestellt wird, enthält beispielsweise 15 bis 45 %, vorzugsweise 20 bis 40 % besonders bevorzugt 25 bis 35 % Proteoglykane im Bezug auf das Trockengewicht des funktionalen Fusionsgewebes. Der Proteoglykangehalt der Fusionsgewebe, die nach dem erfindungsgemäßen Verfahren aus humanen Chondrozyten hergestellt werden, beträgt nach quantitativer Bestimmung beispielsweise 150 bis 550 pg, vorzugsweise 170 bis 500 µg oder 200 bis 460 µg Proteoglykane pro mg Fusionsgewebe.

Funktionales Fusionsgewebe, das nach dem erfindungsgemäßen Verfahren aus humanen Chrondrozyten hergestellt wird, enthält Kollagen Typ II als eines der wichtigsten Bestandteile der extrazellulären Matrix im Gelenkknorpelgewebe und wird in mindestens 70 bis 99 %, vorzugsweise 75 bis 97 %, besonders bevorzugt in 80 bis 95% der Gewebeschnittfläche von Fusionen exprimiert. Die prozentualen Anteile ergeben sich nach computergestützter Auswertung oder Berechnung der positiven Immunfluoreszenz für das Protein Kollagen Typ II bezogen auf die gesamte Gewebeschnittfläche der Fusionsgewebe.

Funktionales Fusionsgewebe, das nach dem erfindungsgemäßen Verfahren aus humanen Chrondrozyten hergestellt wird, enthält beispielsweise 80 bis 99 % oder mehr, vorzugsweise 85 bis 98 % oder 80 bis 98 %, besonders bevorzugt 85 bis 97 % oder 90 bis 96 % oder 95 % Kollagen Typ II bezogen auf den Gesamtkollagengehalt des funktionalen Fusionsgewebes.

Natives Gelenkknorpelgewebe des Menschen enthält etwa 1 bis 5% Knorpelzellen (Chondrozyten) im Bezug auf das Gewebevolumen. Die Knorpelzellen sind dabei die essentiellen Produzenten der Matrixmoleküle des funktionalen nativen Gewebes.

Funktionales Fusionsgewebe, das nach dem erfindungsgemäßen Verfahren aus humanen Chrondrozyten hergestellt wird, enthält beispielsweise 0,1 bis 10 % oder 0,2 bis 8 %, vorzugsweise 0,4 bis 6 % oder 0,5 bis 5 %, besonders bevorzugt 0,7 bis 3 % oder 0,9 bis 1 % Chrondrozyten, die die extrazellulären Matrixmoleküle produzieren. Das funktionale Knorpelgewebe enthält damit mindestens 70 bis 90 %, vorzugsweise mindestens 80 % EMZ.

Eine Ausführungsform der Erfindung betrifft ein erfindungsgemäßes Verfahren, dadurch gekennzeichnet, dass 5 bis 10 oder mehr, vorzugsweise 6 oder 7, besonders bevorzugt 8 oder 9 Sphäroide fusioniert werden. In einer besonders bevorzugten Ausführungsform des Verfahrens werden 5 Sphäroide zur Herstellung des funktionalen Fusionsgewebes fusioniert.

Mit dem Verfahren kann funktionales Fusionsgewebe unterschiedlicher Größe hergestellt werden. Einzelne Sphäroide haben beispielsweise einen Durchmesser von ca. 750 - 1500 µm, erfindungsgemäß von 800 - 1400 µm, besonders bevorzugt von 1000 - 1300 µm. Entsprechend der Anzahl der fusionierten Sphäroide ergibt sich daraus auch die Größe des funktionalen Fusionsgewebes. Die Größe des funktionalen Fusionsgewebes hängt neben der Anzahl auch von der Selbstanordnung (Form) der fusionierten Einzelsphäroide ab. Im erfindungsgemäßen Fall von mindestens 5 Einzelsphäroiden in der Fusion könnte man die Maße in etwa mit 2000 - 3000 µm x 3000 - 4000 µm angeben. Bei mehr als 5 Einzelsphäroiden sind die Maße entsprechend größer. Die Größe kann beispielsweise histologisch bestimmt werden. Die entsprechenden Methoden sind dem Fachmann bekannt.

Dabei ist die spezielle Anordnung der dedifferenzierten Zellen an der Oberfläche von Einzelsphäroiden entscheidend sowie das Vorhandensein mindestens eines Differenzierungsinduktors zur Herstellung von funktionalen Fusionsgewebe. Die enge räumliche Anordnung von 5 oder mehr Sphäroiden stellt einen Differenzierungsinduktor dar. Die Nähe mehrerer Sphäroide zueinander führt dabei nicht nur zur Koaleszenz (Verschmelzung) mehrerer kleinerer Zellaggregate (Sphäroide) sondern induziert auch die Redifferenzierung der dedifferenzierten Zellen in den Sphäroiden und dem daraus gebildeten Fusionsgewebe. Deshalb werden mit dem erfindungsgemäßen Verfahren nicht nur größere Mikrogewebe erzeugt, sondern auch Mikrogewebe, die redifferenzierte Zellen enthalten und in ihrer biologischen Funktionalität dem nativen Gewebe ganz oder weitgehend entsprechen.

Ein erfindungswesentliches Merkmal ist deshalb die besondere 3D-Umgebung (auch besondere 3D-Kultur genannt) der Sphäroide bei der Herstellung des funktionalen Fusionsgewebes. "3D-Umgebung" bedeutet, dass eine einzelne Zelle in jeder Richtung mit einer anderen Zelle Kontakt aufnehmen kann. Die kugelartigen Sphäroide werden in einer besonderen "3D-Umgebung" auf engem Raum in Kontakt gebracht. Durch die dadurch mögliche Kontaktaufnahme der Zellen im Randbereich der einzelnen Sphäroide mit den Zellen im Randbereich der anderen Sphäroide bilden sich Fusionsgewebe, d.h. es können größere Gewebestücke hergestellt werden. Die besondere 3D-Umgebung wird erfindungsgemäß vorzugsweise dadurch erzeugt, dass die Sphäroide sich spontan und selbstständig in eine Position bringen, in der sie ideal fusionieren können. Die ideale Position ist beispielsweise dadurch gekennzeichnet, dass sich Zell-Zell-Kontakte (Tight Junctions, Desmosomen und ähnliches) ausbilden können und die extrazelluläre Matrix-Bildung induziert wird, wobei die gebildete EMZ die organotypischen Eigenschaften im Hinblick auf die Expressionsmuster aufweist. Die besondere 3D-Umgebung bildet sich dann aus, wenn 5 oder mehr Sphäroide mit einem Durchmesser von mindestens 800 µm fusioniert werden. Durch Fusion von 5 oder mehr Sphäroiden mit einem Durchmesser von mindestens 800 µm wird die Bildung von funktionalem Fusionsgewebe induziert.

In einer Ausführungsform werden die Einzelsphäroide, erfindungsgemäß mindestens 5 Einzelsphäroide mit einem Durchmesser von mindestens 800 µm, auf eine konkave Kultivierungsoberfläche aufgebracht, damit diese beim Aufbringen auf die Fläche selbständig (spontan) die ideale Position, d.h. Nähe zueinander einnehmen, um fusionieren zu können. In einer Ausführungsform der Erfindung wird die konkave Oberfläche durch ein Verfahren bereitgestellt, das die Schritte umfasst: a) Agarose wird im Zellkulturmedium gelöst und b) ein definiertes Volumen, beispielsweise 100 µl, in ein Kulturgefäß, beispielsweise in ein Well einer 96-Well-Platte, möglichst heiß gegossen. Beim Abkühlen wird die Agarose fest. Dies geschieht zeitlich versetzt von den Rändern zur Mitte der Kultivierungsschale hin, wodurch sich die Agaroseoberfläche an den Seiten des Wells "hochzieht" und sich insgesamt eine ideale konkave Oberfläche ausbildet. Andere geeignete konkave Flächen lassen sich entsprechend herstellen.

In einer Ausführungsform der Erfindung werden die Einzelsphäroide, erfindungsgemäß mindestens 5 Einzelsphäroide mit einem Durchmesser von mindestens 800 µm, auf eine Kultivierungsoberfläche aufgebracht, die so strukturiert ist, dass die Sphäroide vorzugsweise selbständig (spontan) die ideale Position, d.h. Nähe zueinander einnehmen, um fusionieren zu können. Dabei ist die Kultivierungsoberfläche so strukturiert, dass die Sphäroide nicht bevorzugt mit der Kultivierungsoberfläche sondern bevorzugt miteinander in Wechelswirkung treten. Eine entsprechend strukturierte, geeignete Kultivierungsoberfläche ist beispielsweise eine hydrophobe Oberfläche wie beispielsweise Agarose.

Das erfindungsgemäße Verfahren wird vorzugsweise in vitro durchgeführt, um in vitro Mikrogewebe (funktionales Fusionsgewebe) herzustellen. Dieses kann beispielsweise als Transplantat, Implantat, Zubereitung, beispielsweise Gewebezubereitung, Arzneimittel oder Nahrungsmittel oder für Testsysteme verwendet werden.

Gegenstand der Erfindung ist deshalb ein Verfahren zur Herstellung von funktionalem Fusionsgewebe unter Verwendung eines speziellen Kultursystems zusammen mit Faktoren, die die Differenzierung fördern, aber ohne Zuhilfenahme von Gerüstmaterial. Das Verfahren umfasst zwei Aggregationsschritte: Im ersten Aggregationsschritt werden aus den isolierten Zellen, die teilweise oder ganz dedifferenziert sein können, einzelne isolierte Sphäroide hergestellt, die dann im zweiten Aggregationsschritt der Fusionskultivierung in Anwesenheit von Komponenten, die für die Zellkondensation und Zellkommunikation relevant sind (im Rahmen dieser Erfindung "Differenzierungsinduktor" genannt) weiter fusioniert werden.

Erfindungsgemäß werden im zweiten Aggregationsschritt mehrere Schichten der Oberflächen von in-vitro-Geweben, insbesondere von Sphäroiden in engem Kontakt zueinander angeordnet (erfindungsgemäß "Kultivierung in 3D-Umgebung") und dadurch die mesenchymale Kondensation nachgeahmt. Der Differenzierungsinduktor ist dabei die Zell-Zell- und/oder Zell-Matrix-Wechselwirkung und / oder die Ausbildung von Gap Junctions.

Erfindungsgemäß umfasst ist sowohl die Kombination von mehreren einzelnen Sphäroiden mit bereits fusionierten Geweben oder die Kombination von mehreren einzelnen Sphäroiden.

Eine weitere Ausführungsform der Erfindung betrifft ein erfindungsgemäßes Verfahren, dadurch gekennzeichnet, dass mindestens 1 x 10⁵ Zellen / Well einer Mikrotiterplatte, vorzugsweise 3 x 10⁵ Zellen / Well, besonders bevorzugt 5 x 10⁵ Zellen / Well oder mehr fusioniert werden.

"Differenzierungsinduktoren" im Sinne dieses Verfahrens sind vorzugsweise mechanische und/oder chemische und/oder biochemische Differenzierungsinduktoren. Als mechanische Differenzierungsinduktoren sind beispielsweise die Kultivierung in 3D-Umgebung mit mindestens 5 Shäroiden, die Kultivierung in Gegenwart von mechanischen Stimuli wie Druckapplikation, Kultivierung in Bioreaktoren wie Roting Wall Vessel oder Spinner Flaschen geeignet. Als chemische Differenzierungsinduktoren sind beispielsweise Ascorbinsäure, insbesondere L-Ascorbinsäure und Derivate von Ascorbinsäure wie Ascorbat-2-phosphat geeignet. Als biochemische Differenzierungsinduktoren sind beispielsweise Proteine wie Proteine der TGF-β Superfamilie beispielsweise die TGF-β Isoformen (TGF-β1, TGF-β2, TGF-β3) und Bone Morphogenetic Proteins (BMP-2, BMP-3, BMP-4, BMP-5, BMP-6, BMP-7), Growth Differentiation Factor wie beispielsweise GDF-5 und GDF-10, Insulin-like growth factor wie IGF-1 geeignet. Grundsätzlich sind alle differenzierungsmodulierenden Substanzen als Differenzierungsinduktoren geeignet, beispielsweise auch Glucocorticoide wie Dexamethason. Differenzierungsinduktoren im Sinne der vorliegenden Erfindung sind alle Komponenten und Faktoren, die für die Zellkondensation und Zellkommunikation relevant sind oder die Zellkondensation und Zellkommunikation beeinflussen. Erfindungsgemäß können ein oder mehrere Differenzierungsinduktoren in dem Verfahren eingesetzt werden. Die Brauchbarkeit einzelner Differenzierungsinduktoren sowie die Eignung von Kombinationen von mehreren Differenzierungsinduktoren kann vom Fachmann beispielsweise anhand der Expressionsmuster von Kollagen Typ II, gewebespezifischen Proteoglykanen, Kollagen Typ I und/oder S100 Protein überprüft werden.

Eine bevorzugte Ausführungsform der Erfindung betrifft ein erfindungsgemäßes Verfahren, dadurch gekennzeichnet, dass der/die weiteren Differenzierungsinduktor(en) ausgewählt werden aus den Differenzierungsinduktoren TGF-β zusammen mit Ascorbinsäure, insbesondere TGF-β2 zusammen mit L-Ascorbinsäure.

Eine weitere Ausführungsform der Erfindung betrifft ein erfindungsgemäßes Verfahren dadurch gekennzeichnet, dass das solide Gewebe, aus dem die Zellen isoliert werden, humanen oder tierischen Ursprungs ist. Das solide Gewebe kann ento-, ekto- und/oder mesodermalen Ursprungs sein. Mesenchymale Stammzellen (MSC) besitzen ein hohes Proliferations- und Differenzierungspotential. Adulte mesenchymale Stammzellen tragen zur Aufrechterhaltung und Regeneration des Stütz- und Bindegewebes, wie Knochen, Knorpel, Muskel, Bändern, Sehnen und Fettgewebe bei. Darüber hinaus unterstützen sie Wachstum und Entwicklung der Vorläuferzellen des Blutes im Knochenmark. MSC aus unterschiedlichen Geweben (Knochenmark, Knorpel, Fettgewebe, Muskel, Lebergewebe, Blut, Amnionflüssigkeit) lassen sich kultivieren und in vitro in unterschiedliche Gewebe ausdifferenzieren.

Das Verfahren zur Herstellung von funktionalem Fusionsgewebe ermöglicht, einzelne Zellaggregate zu einem größeren "knorpelähnlichen" Gewebe zu vereinen, welches dann auch wesentlich verbesserte Eigenschaften hinsichtlich der Differenzierung und der Gewebequalität aufweist, was schließlich eine Anwendung beispielsweise als in vitro Testsystem für die pharmazeutische Industrie oder als Transplantat in der regenerativen Medizin begünstigt.

Im Rahmen des erfindungsgemäßen Verfahrens werden vorzugsweise tierische, insbesondere humane Zellen verwendet. Die Zellen sind vorzugsweise adulte Zellen. Die Zellen sind beispielsweise im Zellzyklus arretierte Zellen. Vorzugsweise werden frisch isolierte Zellen verwendet. Eine weitere Ausführungsform der Erfindung betrifft ein erfindungsgemäßes Verfahren dadurch gekennzeichnet, dass die Zellen frisch isolierte und/oder postmitotische tierische oder humane Zellen sind. In einer bevorzugten Ausführungsform der Erfindung werden adulte humane Chondrozyten, insbesondere frisch isolierte postmitotische Chondrozyten (Knorpelzellen) verwendet. In einer weiteren bevorzugten Ausführungsform der Erfindung werden adulte humane Knochenzellen, insbesondere Osteozyten und/oder Osteoblasten verwendet.

Eine weitere Ausführungsform der Erfindung betrifft ein erfindungsgemäßes Verfahren dadurch gekennzeichnet, dass mehrere verschiedene Zellen verwendet werden, um komplexe funktionale Gewebe herzustellen. Beispielsweise können Zellen verwendet werden, die Chrondrozyten umfassen.

Eine weitere Ausführungsform der Erfindung betrifft ein erfindungsgemäßes Verfahren dadurch gekennzeichnet, dass dedifferenzierte Zellen durch Kultur in Monolayer hergestellt werden.

Die Kultivierung der isolierten Zellen, beispielsweise in Verfahrensschritt b), dient der Vermehrung der isolierten Zellen. Die Zellen werden dazu aus dem Gewebe isoliert und in eine neue Umgebung gebracht, in der sie sich ideal vermehren. Entsprechende Methoden sind dem Fachmann bekannt. Durch das Einbringen von isolierten Zellen in die neue Umgebung kommt es zur Dedifferenzierung der isolierten Zellen. Durch die Dedifferenzierung verändert sich die Funktionalität der Zellen, was beispielsweise durch die veränderten Expressionsmuster von Kollagen Typ II, Kollagen Typ I, gewebespezifischen Proteoglykanen und/oder S100 Protein nachgewiesen werden kann. Eine Ausführungsform der Erfindung betrifft ein erfindungsgemäßes Verfahren, wobei die isolierten Zellen dadurch vermehrt werden, dass sie in Monolayerkultur kultiviert werden, beispielsweise für zwei oder mehr Passagen.

Gegenstand der Erfindung ist ein Verfahren zur Herstellung von funktionalem Fusionsgewebe umfassend die Schritte
a) Expansion isolierter Zellen, insbesondere von Chondrozyten in einer Monolayerkultur unter Dedifferenzierung der Zellen,
b) Aggregation der isolierten und dedifferenzierten Zellen zu Sphäroiden,
c) Aggregation der Sphäroide unter Redifferenzierung der Zellen durch Kultivierung in einer 3D-Umgebung (Fusionskultivierung) gegebenenfalls in Gegenwart weiterer Differenzierungsinduktoren wie beispielsweise TGF-β2 und / oder L-Ascorbinsäure.

Eine weitere Ausführungsform der Erfindung betrifft ein erfindungsgemäßes Verfahren dadurch gekennzeichnet, dass das Verfahren einen weiteren Schritt umfasst, in dem die Bildung von Sphäroiden angeregt wird. Dazu werden die isolierten bzw. vermehrten bzw. dedifferenzierten Zellen in eine Umgebung gebracht in der sich die Zellen bevorzugt an andere Zellen und nicht an die Oberfläche der Umgebung anheften. Dazu können beispielsweise Gefäße mit einer hydrophoben Oberfläche für die Kultivierung verwendet werden. Eine weitere Ausführungsform der Erfindung betrifft deshalb ein Verfahren, wobei dedifferenzierte Zellen im Anschluss an die Dedifferenzierung auf einer hydrophoben Oberfläche, vorzugsweise auf einer Agaroseschicht, kultiviert werden. Die Kultivierung auf Agarose regt die Sphäroidbildung an.

Eine weitere Ausführungsform der Erfindung betrifft ein erfindungsgemäßes Verfahren dadurch gekennzeichnet, dass die dedifferenzierten Zellen im Anschluss an die Dedifferenzierung für 1 bis 5 Tage, vorzugsweise zwei Tage auf Agarose oder einer anderen hydrophoben Oberfläche kultiviert werden.

Der Nachweis der Dedifferenzierung und Redifferenzierung von Chrondrozyten kann über S100, Kollagen Typ II, Kollagen Typ I und gewebespezifische Proteoglykane erfolgen. Die Dedifferenzierung der Zellen zeichnet sich dadurch aus, dass S100, Kollagen Typ II, gewebespezifische Proteoglykane vermindert exprimiert werden und Kollagen Typ I verstärkt exprimiert wird. Die Differenzierung nativer Zellen und die Differenzierung funktionalen Fusionsgewebes zeichnen sich durch eine hohe Expression von Kollagen Typ II, gewebespezifischen Proteoglykanen, S100 Protein aus und durch eine niedrige Expression von Kollagen Typ I.

Für die Kultivierung werden erfindungsgemäß übliche Kulturmedien vorzugsweise ohne Zusatz von Antibiotika oder Fungistatika verwendet. Dem Kulturmedium wird vorzugsweise noch Serum zugesetzt, beispielsweise Humanserum in einer Konzentration von etwa 1 % bis 20 %, vorzugsweise 5 bis 10 %. Erfindungsgemäß geeignet sind alle üblichen Kulturmediem, beispielsweise HAMs, Alpha-Medium, DMEM, MEM. Eine besondere Ausführungsform des erfindungsgemäßen Verfahrens betrifft die Verwendung von Alpha-Medium und HAMs F12 (1:1) als Kulturmedium, wobei vorzugsweise L-Glutamin und Humanserum zugesetzt werden.

Die Fusion der Sphäroide in dem erfindungsgemäßen Verfahren erfolgt beispielsweise für 3 bis 7 Wochen, vorzugsweise 4 oder 5 Wochen, besonders bevorzugt 6 Wochen. Die Fusion, z.B. gemäß Schritt d) des Verfahrens, wird in einer besonderen Ausführungsform mit 5 Sphäroiden / Well einer Mikrotiterplatte und bevorzugt in Gegenwart von TGF-β2 und gegebenenfalls L-Ascorbinsäure durchgeführt.

Gegenstand der Erfindung ist auch funktionales Fusionsgewebe erhältlich durch ein erfindungsgemäßes Verfahren.

Eine Ausführungsform der Erfindung betrifft funktionales humanes Knorpelgewebe, das erhalten wird, wenn in dem erfindungsgemäßen Verfahren humane Chrondrozyten als Zellen eingesetzt werden. Das funktionale Knorpelgewebe ist dadurch gekennzeichnet, dass der Kollagenanteil 50 bis 70 % und der Proteoglykananteil 15 bis 45 % in Bezug auf das Trockengewicht des funktionalen Knorpelgewebes beträgt. Das funktionale Knorpelgewebe ist weiterhin dadurch gekennzeichnet, dass der Anteil des Kollagen Typ II 85 bis 98 % bezogen auf den Gesamtkollagengehalt des funktionalen Knorpelgewebes beträgt. In einer anderen Ausführungsform des Verfahrens wird Kollagen Typ II in mindestens 80 - 95 % der Gewebeschnittflächen von Fusionen exprimiert, bezogen auf die gesamte Gewebeschnittfläche des funktionalen humanen Knorpelgewebes.

Die Erfindung umfasst beispielsweise funktionales muskoskelletales Gewebe, funktionales Skelettgewebe, funktionales Knorpelgewebe, funktionales Knochengewebe, funktionales Meniskusgewebe, funktionales Epithelgewebe, funktionales Bindegewebe, funktionales Stützgewebe, funktionales Muskelgewebe, funktionale glatte Muskulatur, funktionale Herzmuskulatur, funktionales Nervengewebe, funktionales Funktionsgewebe (Parenchym), funktionales Zwischengewebe (Interstitium), funktionales Organgewebe, beispielsweise funktionales Lebergewebe, funktionales Nierengewebe, funktionales Nebennierenrindengewebe, funktionales Magengewebe, funktionales Bauchspeicheldrüsengewebe, funktionales Herzgewebe, funktionales Lungengewebe, funktionales Hautgewebe, funktionales Augenhornhautgewebe, funktionales subkutanes Gewebe, funktionales Gewebes des Verdauungstrakts, funktionales Knochenmark, funktionales Gewebe des Gehirns, funktionales Schilddrüsengewebe, funktionales Milzgewebe, funktionales Gelenkgewebe, funktionales Sehnengewebe, das nach dem erfindungsgemäßen Verfahren erhältlich ist.

Beispielsweise umfasst die Erfindung nach dem Verfahren hergestelltes funktionales Fusionsgewebe, das ein autologes, xenogenes, allogenes oder syngenes Fusionsgewebe im Hinblick auf den betreffenden Spender und Empfänger, Mensch oder Tier, des für das Verfahren eingesetzten Gewebes bzw. der eingesetzten Zellen ist. Die Erfindung umfasst insbesondere autologes, xenogenes, allogenes oder syngenes funktionales Knorpel- und Knochengewebe.

Die durch das erfindungsgemäße Verfahren erhaltenen Fusionsgewebe sind im Vergleich zu den im Stand der Technik, insbesondere in DE 100 13 223 beschriebenen, nicht nur größer, sondern auch in ihrer Funktionalität verschieden. Die erfindungsgemäßen Fusionsgewebe sind aus redifferenzierten Zellen aufgebaut, sie sind funktional und in ihrer Struktur den nativen Geweben, aus denen die verwendeten Zellen isoliert wurden, ähnlicher bzw. sehr ähnlich bzw. identisch.

Im Gegensatz zu dem in US 7 887 843 B2 erzeugten Gewebe werden in dem erfindungsgemäßen Verfahren mehr Zellen über einen kürzeren Zeitraum kultiviert, um Sphäroide herzustellen. Die dadurch erhalten Sphäroide sind größer und überraschenderweise in ihrer Funktionalität den nativen Geweben ähnlicher. Das nach dem erfindunggemäßen Verfahren aus Chrondrozyten hergestellte Fusionsgewebe besteht aus 0,1 bis 10 % EZMproduzierenden Zellen und somit aus 90 bis 99,9 % EZM. Die mit dem in US 7 887 843 B2 beschrieben Verfahren hergestellten Fusionsgewebe unterscheiden sich von den erfindungsgemäßen Fusionsgeweben hinsichtlich ihrer Funktionalität und der Expressionsmuster von Kollagen Typ I, Kollagen Typ II und gewebespezifischen Proteoglykanen.

Gegenstand der Erfindung ist auch die Verwendung von funktionalem Fusionsgewebe das nach dem erfindungsgemäßen Verfahren erhältlich ist, beispielsweise als Implantat, Transplantat, funktionales Ersatzgewebe, zur in vitro Gewebekultivierung, zur Herstellung von größeren transplantierbaren Geweben, zur Herstellung von in vitro und in vivo Geweben, zum Tissue Engineering. Insbesondere betrifft die Erfindung die Verwendung bzw. spezifische Anwendung des erfindungsgemäßen Fusionsgewebes als autologes, xenogenes, allogenes oder syngenes Implantat, Transplantat oder Gewebezubereitung.

Gegenstand der Erfindung ist ein Implantat, Transplantat und funktionales Ersatzgewebe erhältlich nach dem erfindungsgemäßen Verfahren. Das in vitro oder in vivo hergestellte Implantat, Transplantat oder funktionale Ersatzgewebe kann in das umliegende native Gewebe des Empfängers eingebracht werden. In dem nach dem erfindungsgemäßen Verfahren hergestellten funktionalen Fusionsgewebe, d.h. auch in dem Implantat, Transplantat oder funktionalem Ersatzgewebe findet keine Zellteilung statt. Wird das funktionale Fusionsgewebe in natives Gewebe eingebracht, dann heften sich die Zellen des funktionalen Fusionsgewebes an das native Gewebe an (Adhäsion) und wandern in die Lücken ein (Migration) ohne sich jedoch zu teilen. Dadurch ist eine optimale Versorgung/Wiederherstellung gewährleistet, jedoch ohne das Risiko einer unkontrollierten Proliferation des funktionalen Ersatzgewebes.

Alternativ können die mindestens 5 Sphäroide mit einer Größe von mindestens 800 µm an die Stelle gebracht werden, wo das Implantat, Transplantat oder funktionale Ersatzgewebe im Empfänger lokalisiert sein soll und die Fusion in vivo durchgeführt werden. Idealerweise wird dazu eine entsprechende konkave Stelle an der Empfängerstelle vorbereitet.

Gegenstand der Erfindung ist auch die Verwendung / spezifische Anwendung des erfindungsgemäßen funktionalen Fusionsgewebes zusammen mit Sphäroiden.

Gegenstand der Erfindung ist auch eine Zubereitung, insbesondere eine pharmazeutische Zubereitung oder eine Gewebezubereitung, ein Arzneimittel, ein Transplantat oder ein Implantat bestehend aus oder enthaltend funktionales Fusionsgewebe, dass nach dem erfindungsgemäßen Verfahren erhältlich ist und gegebenenfalls weitere Hilfs- und Zusatzstoffe.

Gegenstand der Erfindung sind auch pharmazeutische Zubereitungen, Gewebezubereitungen und Arzneimittel, insbesondere Suspensionen und Lösungen, insbesondere Injektionslösungen, die das erfindungsgemäße funktionale Fusionsgewebe und ggf. weitere Hilfs- und Zusatzstoffe enthalten.

Gegenstand der Erfindung ist auch die spezifische therapeutische/pharmazeutische Anwendung einer erfindungsgemäßen pharmazeutischen Zubereitung, einer Gewebezubereitung, eines erfindungsgemäßen Arzneimittels, eines erfindungsgemäßen Transplantats oder Implantats zur Behandlung von Knorpeldefekten und / oder Knochendefekten, insbesondere traumatischen Knorpeldefekten und / oder Knochendefekten, Läsionen, insbesondere traumatischen Läsionen, bei Knorpeldegenerationen, Knochendegenerationen, Osteoarthritis, zur therapeutische Knorpel- und / oder Knochenregeneration in vivo.

Gegenstand der Erfindung ist auch ein Verfahren zur in vitro Herstellung von Nahrungsmitteln umfassend die Fusion von mindestens 5 Sphäroiden mit einem Durchmesser von mindestens 800 µm. Gegenstand der Erfindung ist auch das dadurch erhältliche Nahrungsmittel.

Gegenstand der Erfindung ist auch die Verwendung des erfindungsgemäßen Fusionsgewebes zum Testen von Wirkstoffen, beispielsweise um neue Wirkstoffe zu Screenen, bekannte Wirkstoffe zu verbessern oder zu validieren oder um für einen bekannten Wirkstoff neue Indikationen und Anwendungsgebiete zu erschließen oder im Rahmen der vorklinischen und klinischen Prüfung zur Datengenerierung und Prüfung von Wirkstoffen oder zur Testung von Schadstoffen.

Gegenstand der Erfindung ist ein Testsystem umfassend funktionales Fusionsgewebe und gegebenenfalls weitere Zusatz- und/oder Hilfsstoffe. Gegenstand der Erfindung ist ein Testsystem, beispielsweise ein Test Kit, umfassend
a) funktionales Fusionsgewebe, eine entsprechende Zubereitung, ein entsprechendes Arzneimittel, ein entsprechendes Transplantat oder Implantat,
b) gegebenenfalls weitere Hilfs- und Zusatzstoffe und
c) Mittel zum Nachweis.

Gegenstand der Erfindung ist beispielsweise auch ein in vitro Testsystem für die experimentelle Pharmakologie. Mit einem solchen Testsystem können neue Wirkstoffe identifiziert und neue und bekannte Wirkstoffe getestet werden, beispielsweise der Effekt eines Arzneimittels auf Knochen und/oder Knorpel. Desweiteren kann der Effekt von Substanzen, künstlichen oder natürlichen Ursprungs auf beispielsweise Knochen und/oder Knorpel getestet werden, beispielsweise von Lebensmitteln, Naturstoffen, Lösungsmitteln, Polymeren etc.. Ein solches Testsystem kann damit beispielsweise Aufschluß über die Toxizität und mögliche Nebenwirkungen liefern und auch zur Bestimmung von Grenzwerten und synergistischen Effekten von Substanzen verwendet werden.

Gegenstand der Erfindung ist deshalb auch ein Verfahren zum Testen von zu untersuchenden Substanzen umfassend die Schritte
a) Herstellung von funktionalem Fusionsgewebe oder eines Testsystems, das funktionales Fusionsgewebe umfasst;
b) das Inkontakt bringen einer zu testenden Substanz mit dem funktionalen Fusionsgeweben aus a),
c) Bestimmung/Nachweis des Effekts der zu untersuchenden Substanz auf das funktionale Fusionsgewebe.

Gegenstand der Erfindung ist auch ein Verfahren zum Testen von zu untersuchenden Substanzen dadurch gekennzeichnet, dass
a) funktionales Fusionsgewebe, eine entsprechende Zubereitung, ein entsprechendes Arzneimittel, ein entsprechendes Transplantat oder Implantat,
b) mit einer oder mehreren zu untersuchenden Substanzen in Kontakt gebracht wird,
c) der Effekt von der/den zu untersuchenden Substanz(en) auf das funktionales Fusionsgewebe, die Zubereitung, das Arzneimittel, das Transplantat oder Implantat nachgewiesen wird.

Im Stand der Technik ist der Einsatz der Fusionskultivierungstechnik zur gezielten, verstärkten Stimulation der Differenzierung von menschlichen Zellen verschiedensten Ursprungs, vor allem Zellen sämtlicher muskoskelettaler Gewebe (nicht nur Knorpelzellen) nicht bekannt. In DE 100 13 223 und Anderer und Libera, 2002 wurde die Vereinigung von zwei einzelnen Sphäroiden zu einem größeren Aggregat offenbart, jedoch nicht die Aggregation von vorzugsweise 5 oder mehr Sphäroiden mit einer Größe von mindestens 800 µm (besondere 3D-Umgebung der erfindungsgemäßen Fusionskultivierungstechnik) mit dem Hintergrund, dadurch die Redifferenzierung der Zellen zu erreichen, die mit einer gesteigerten Produktion knorpelspezifischer extrazellulärer Matrixbestandteile wie Kollagen II und Proteoglykanen einhergeht. Die im Stand der Technik offenbarte Aggregation diente vielmehr dazu, die Generierung eines größeren Zellaggregates zu ermöglichen.

Die vorliegende Erfindung zur Herstellung von Geweben durch Koaleszenz (Verschmelzung) von mindestens5 kleineren Zellaggregaten stellt gezielt größere, klinisch anwendbare Gewebe zur Verfügung, deren Struktur und charakteristische Proteinausstattung dem menschlichen Originalgewebe sehr ähnlich ist. Die spezielle Anordnung der Zellen an der Oberfläche von Einzelsphäroiden und die resultierenden Zell-Zellkontakte bei Berührung mit benachbarten Sphäroiden werden dabei als neue Differenzierungsindukturen (Verstärker) genutzt. Dabei besteht ein wesentlicher Anspruch dieser neuen Technik zur in vitro Gewebekultivierung in der Bereitstellung von größeren transplantierbaren Geweben die sich am Vorbild des jeweiligen natürlichen Körpergewebes (beispielsweise am Gelenkknorpel) orientieren. Diese Technik ist auf alle anderen Gewebe übertragbar und damit universell einsetzbar.

Die vorliegende Erfindung ermöglicht es, Knorpeldefekte verschiedener Größe z.B. im Kniegelenk mit einer geringeren Anzahl größerer, besser differenzierter und funktionaler in vitro Gewebe auszukleiden und so zu regenerieren. Ein weiterer wichtiger Aspekt des erfindungsgemäßen funktionalen Fusionsgewebes ist, dass mit dem erfindungsgemäßen Verfahren der Fusionskultur eine Möglichkeit gefunden werden konnte, ohne den Einsatz differenzierungsfördernder Faktoren wie TGF-β, beachtliche Knorpeleigenschaften des nativen Gewebes in den funktionalen in vitro Geweben zu erzeugen. Zum Nachweis wurde die Fusionskultur mit biochemischen Stimuli kombiniert, um einen synergistischen Effekt hinsichtlich der knorpelspezifischen Differenzierung zu zeigen. Die Fusionskultur induziert im Basalmedium ohne jeglichen Zusatz von Wachstumsfaktoren, Ascorbinsäure oder den Einsatz synthetischer Gerüstmatrices die Differenzierung der Knorpelzellen im funktionalen Fusionsgewebe effektiv und ist deshalbden einzelnen Zellaggregaten (einzelnen Sphäroiden) deutlich überlegen. Das erfindungsgemäße Verfahren ermöglicht die Herstellung einer neuen Generation transplantierbarer Gewebe für eine therapeutische Anwendung mit und ohne zusätzliche Wachstumsfaktoren.

Eine andere Anwendungsmöglichkeit betrifft die Verwendung der in vitro hergestellten funktionalen Fusionsgewebe als Basis für eine Plattformtechnologie zum Testen von Substanzen beispielsweise in der pharmazeutischen Industrie, der chemischen industrie, der Lebensmittelindustrie. Beispielsweise könnte damit im Bereich der rheumatischen Erkrankungen ein Testsystem bereitgestellt werden um die Physiologie von Knorpelzellen in gesunden und in arthrotischinduzierten Gewebeimitaten zu analysieren und zu vergleichen, insbesondere auch im Hinblick auf das Ansprechen auf vermeintlich therapeutische Substanzen.

Die Generierung körperähnlicher Gewebe durch die Fusion einzelner Zellaggregate ist in vitro mit dem erfindungsgemäßen Verfahren möglich. Es konnte gezeigt werden, dass die isolierten Chondrozyten aus verschiedenen Patienten nach der De-Differenzierungsphase im Monolayer allein durch die Fusionskultur ohne den Zusatz bioaktiver Stimulanzien wieder re-differenzieren. Dadurch eröffnet sich auch ein Lösungsansatz für das vielfach auftretende Problem der Donorabhängigkeit, so dass Gewebe mit replizierbaren Eigenschaften und ähnlicher Qualität aus Zellen verschiedener Spender generiert werden können.

Die Verschmelzung mehrerer Zellaggregate (Sphäroide) ermöglicht zu verschieden großen Geweben eine deutlich vielfältigere Formmodellierung, so dass die vorzugsweise in vitro gebildeten funktionalen Fusionsgewebe beispielsweise ideal auf die entsprechende Größe und Form des jeweiligen Defekts angepasst und auch spezielle Formen wie z.B. Meniskusgewebe konstruiert werden könnten. Um die Gewebefunktionalität und die Qualitätsmerkmale des funktionalen Fusionsgewebes weiter zu verbessern, können andere bekannte Methoden zur Stimulation der Differenzierung ggf. zusätzlich eingesetzt werden. Dazu zählt, insbesondere für Knorpelgewebe, die mechanische Stimulation z.B. über Druckapplikation (Kompression und Dekompression) oder die Kultivierung in speziellen Bioreaktoren und Spinner Flaschen, um so die natürlichen Vorgänge und wirkenden Kräfte im Körper, beispielsweise beim Laufen, zu simulieren. Dies sollte einerseits das funktionale Fusionsgewebe optimal mit Nährstoffen versorgen und andererseits auch eine Weiterleitung externer Signale (Mechanotransduktion) gewährleisten, was wiederum die Synthese extrazellulärer Matrixkomponenten anregt.

Besonders in einer Gesellschaft in der die Lebenserwartung der Menschen stetig steigt, sieht sich das Gesundheitswesen zunehmend mit degenerativen Erkrankungen der Gelenke und des gesamten Bewegungsapparats, z.B. durch arthrotische Abbauprozesse, konfrontiert. Somit besteht in Klinik und Forschung ein großer Bedarf an im Labor hergestellten funktionalen Ersatzgeweben. Im bisherigen Stand der Technik haben sich auf diesem Gebiet die erwähnten Zellaggregate in Form von Sphäroiden etabliert, die jedoch in ihrer Regenerationskapazität und der behandelbaren Defektgröße klar eingeschränkt sind. Die nun mit der Fusionskultivierung herstellbaren funktionalen Fusionsgewebe stellen unter den Techniken des "Tissue Engineering"eine Verbesserung gegenüber einzelnen Zellaggregaten (Sphäroiden) und Fusionen mit nur 2 Sphäroiden dar. Mit dem erfindungsgemäßen Verfahren ist es möglich differenzierte, formbare Körpergewebe verschiedener Größe zu generieren. Die Verwendung des funktionalen Fusionsgewebes zur Entwicklung von anwendbaren Geweben zur Nachbildung von Körpermaterialen verschiedenen muskoskelettalen Ursprungs z.B. für den Einsatz in der regenerativen Medizin und in der pharmazeutischen Industrie, zur Untersuchung und zum besseren Verständnis von Erkrankungsmechanismen und als Plattform zum Testen neuer Medikamente.

Im Rahmen dieser Erfindung wurden ein Modell für die In-vitro-Chondrogenese mit Implikationen für sowohl die Grundlagenforschung als auch klinische Ansätze erstellt. Es wurden humane Chondrozyten verwendet, um knorpelartige dreidimensionale In-vitro-Mikrogewebe unter Verwendung der erfindungsgemäßen Fusionskultur zusammen mit die Differenzierung fördernden bioaktiven Molekülen, jedoch ohne Zuhilfenahme jedweden Gerüstmaterials zu erzeugen. Nach der Expansion isolierter Chondrozyten in einer Monolayerkultur, begleitet von der Dedifferenzierung der Zellen, waren adäquate chondrogene Stimuli zur Redifferenzierung erforderlich. Einerseits durch Kultivieren von Chondrozyten in einer 3D-Umgebung und andererseits durch Zugabe von Wachstumsfaktoren, wie transformierendem Wachstumsfaktor-beta-2 (TGF-β2) und L-Ascorbinsäure, als Antioxidans für die Prolylhydroxylase, die für eine einwandfreie Kollagensynthese wichtig ist.

Da die mesenchymale Kondensation einer der frühesten Schritte während der Entwicklung vieler Gewebe, wie Knochen, Muskeln, Nieren oder Knorpel, ist, ahmt die Kultivierung von humanen Chondrozyten in einer 3D-Umgebung in vitro in Form von Sphäroiden diesen Aggregationsprozess von mesenchymalen Zellen als den ersten Schritt der embryonalen Chondrogenese nach. Darüber hinaus stellt die Kombination von mehreren einzelnen Sphäroiden mit fusionierten Geweben als ein zweiter Aggregationsschritt eine neue Methode bereit, die überraschend die Differenzierung von knorpelartigen In-vitro-Geweben fördert. Um die Qualität der erzeugten knorpeligen Konstrukte zu evaluieren, wurden Differenzierungsmarker verwendet. Von diesen Markern ist Kollagen Typ II das kennzeichnende Protein für hyalinen Knorpel, wohingegen die Expression von Kollagen Typ I dazu verwendet wird, dedifferenzierte Regionen, die fibrösem Bindegewebe ähneln, in den In-vitro-Geweben nachzuweisen. Darüber hinaus ist ein hoher Gehalt an Proteoglykanen für funktionalen Knorpel wichtig und auch das kleine intrazelluläre Protein S100 ist ein Indikator für differenzierte Knorpelzellen. Obwohl S100 eher selten als Marker für Chondrozyten verwendet wird, kann es Knorpel- und Chondrozytendifferenzierung demonstrieren, da bekannt ist, dass eine verminderte S100-Expression in humanen Gelenkchondrozyten mit kumulativen Populationsverdopplungen korreliert. Die verschiedenen S100-Proteine, z. B. S100A1 und S100B, sind an einem breiten Spektrum von intrazellulären Prozessen, wie Zell-Zell-Kommunikation, Zellform, Zellstruktur und -wachstum, jedoch auch an der intrazellulären kalziumabhängigen Signaltransduktion beteiligt und sie verhalten sich Zytokinen ähnlich.

Die Chondrozyten wurden für etwa 40 Tage in einer 3D-Umgebung kultiviert, die partiell mit Differenzierungsfaktoren angereichert war. Die konstruierten knorpeligen Mikrogewebe wurden histologisch und immunhistochemisch analysiert, um die Menge und Verteilung von gewebespezifischen Matrixkomponenten zu bestimmen. Da das Vorliegen von Knorpelmatrixmolekülen und wichtigen Markern auf der Proteinebene für die Qualitätsbeurteilung der erzeugten Mikrogewebe von Bedeutung ist, wurden in situ histologische und immunhistochemische Nachweistechniken verwendet. Es wurde die In-vitro-Chondrogenese durch gealterte humane Chondrozyten in der erfindungsgemäßen dreidimensionalen "Zwei-Schritt"-Fusionskultur sowie die Bildung von knorpelartigen Mikrogeweben ohne Verwendung jedweden Gerüsts oder tragenden Gelmaterials demonstriert. Die erfindungsgemäßen Fusionsgewebe sind ein Modellsystem für das Studium sowohl des Metabolismus der betreffenden Zellen, z.B. von Chondrozyten und deren Aktivität in einer dreidimensionalen Konfiguration. Darüber hinaus sind die erzeugten Fusionsgewebe, z.B. Knorpelmikrogewebe bei einer autologen Anwendung als Transplantate geeignet, insbesondere für traumatische Defekte.

Im Zellzyklus arretierte Chondrozyten, die aus Knorpelgewebe isoliert worden waren, begannen erneut, sich in einer Monolayerkultur zu vermehren. Wenn sie als Monolayer serienkultiviert wurden, hörten sie auf, knorpelspezifische Makromoleküle zu synthetisieren und ersetzten sie durch Moleküle, die normalerweise von mesenchymalen Zellen von anderen Bindegeweben exprimiert werden.

Im Stand der Technik ist bekannt, einen chondrozytenspezifischen Phänotyp in verschiedenen Kultursystemen zu konservieren. Gute Ergebnisse wurden erzielt, indem humane Chondrozyten auf biologisch abbaubaren Gerüsten (Scaffolds) gezüchtet wurden. Die meisten synthetischen Polymermatrizes neigen jedoch dazu, sich bei einem deutlich sauren pH-Wert zu zersetzen, was sich als schädlich für implantierte Zellen und umliegende Gewebe bewiesen hat. Des Weiteren ist der Einfluss des Gerüstmaterials auf das Chondrozytenverhalten und die Zell-Zell-Wechselwirkungen oftmals schwer zu unterscheiden. Im Gegensatz zu diesen Ansätzen basiert das erfindungsgemäße Verfahren und die erhaltenen funktionalen Fusionsgewebe auf einer gerüstfreien Induktion von knorpelartigen In-vitro-Geweben, wobei die Nachahmung des ersten Schritts der In-vivo-Chondrogenese ausgenutzt wird. Die Zellen werden nicht in eine feststehende Gerüststruktur gezwängt, was eine bessere Einpassung in einen gegebenen Gelenkknorpeldefekt ermöglichen kann. Im Stand der Technik wird nur die Redifferenzierung von tierischen Chondrozyten durch 3D-Kultursysteme beschrieben. Die Erzeugung von dreidimensionalen Knorpelgewebestrukturen aus adulten humanen Chondrozyten ist jedoch schwieriger. In enormen Zahlen aus gesunden Gelenken von Tieren isolierte Chondrozyten können direkt für 3D-Kulturen verwendet werden, wohingegen In-vitro-Studien mit humanen Chondrozyten in den meisten Fällen Monolayer-Expansionsschritte erfordern. Chondrozyten von verschiedenen Kniegelenken von Tieren unterscheiden sich bereits in Bezug auf ihre In-vitro-Biologie von Zellen, die aus der entsprechenden Oberfläche von Gelenken vom Menschen isoliert wurden.

Die resultierenden einzelnen Sphäroide oder Fusionen bei Verwendung von humanen Chondrozyten stellen eine allmähliche Redifferenzierung von Zellen in diesen Mikrogeweben dar. Dieser Redifferenzierungsprozess wurde biochemisch beeinflusst, indem das Medium mit TGF-β2 supplementiert wurde. In Bezug auf Knorpel wird TGF-β funktional freigesetzt und die entsprechenden Rezeptoren werden ebenfalls in Chondrozyten exprimiert. Eine Reihe von Studien hob die Rolle von TGF-β und insulin-ähnlichem Wachstumsfaktor-I (IGF-I) als wichtige Mediatoren bei der Förderung der Gewebereparatur durch erhöhte Produktion von Hauptkomponenten der Gelenkknorpelmatrix hervor. Die Auswirkung von TGF-β auf den Matrixmetabolismus in Chondrozyten ist jedoch umstritten. Widersprüchliche Berichte haben Anstiege und Abnahmen der Proteoglykansynthese, eine Verstärkung der Differenzierung und eine starke Zunahme oder eine Hemmung des Wachstums demonstriert. In der vorliegenden Erfindung stellte sich TGF-β2 als ein wirksamer Promotor der chondrogenen Differenzierung in den 3D-Kultursystemen heraus.

Eine Kombination von TGF-β2 mit L-Ascorbinsäure in dem Kulturmedium führte in Bezug auf die chondrogene Redifferenzierung in Mikrogeweben zu ähnlichen Ergebnissen. Von der Supplementierung mit Ascorbat wird häufig berichtet, dass sie die Differenzierung begünstigende Effekte im Hinblick auf die chondrogene Zelllinie vermittelt. Die Auswirkung von Ascorbinsäure auf die Proteoglykansynthese durch Chondrozyten wird kontrovers diskutiert. Die Ergebnisse der vorliegenden Erfindung zeigen, dass Ascorbinsäure allein die PG-Synthese nicht stimulieren konnte. Safranin-O-Positivität wurde in Fusionen nachgewiesen, die mit oder ohne Ascorbinsäure kultiviert wurden, was darauf hindeutet, dass nicht Ascorbat, sondern die erfindungsgemäße Fusionskultur als Stimulus induzierend wirkt. Es wird allgemein angenommen, dass Ascorbat die Kollagenproduktion durch seine Auswirkung auf die Prolylhydroxylierung moduliert. Interessanterweise wurde Kollagen Typ II durch Zugabe von L-Ascorbinsäure allein weder in einzelnen Sphäroiden noch in Fusionen vermehrt. Im Gegensatz dazu wurde Kollagen Typ I in einzelnen Sphäroiden, die in ascorbathaltigem Medium kultiviert wurden, im Vergleich zu den in Basalmedium kultivierten, stärker exprimiert. Dieses Phänomen deutet darauf hin, dass L-Ascorbinsäure die Kollagensynthese oder -assemblierung im Allgemeinen anstelle von Kollagen Typ II im Besonderen stimulierte und Ascorbat die Produktion von dem Kollagentyp fördert, auf dessen Expression die Transkriptionsmaschinerie der Zellen programmiert ist. Da in einer Monolayerkultur expandierte, dedifferenzierte Chondrozyten in dem erfindungsgemäßen Verfahren verwendet wurden, kann es sein, dass die Zellen zur Synthetisierung von Kollagen Typ I neigen, die wiederum von der Gegenwart von L-Ascorbinsäure begünstigt wurde. Die Kombination mit TGF-β2 schien diesen Prozess umzukehren, da TGF-β2 die Genexpression von Kollagen Typ II selektiv auslösen kann. Die Proteinexpression in einzelnen Sphäroiden und fusionierten Mikrogeweben veränderte sich in Richtung einer verbesserten chondrogenen Differenzierung, was durch eine erhöhte Expression von Kollagen Typ II und S100 dargestellt wurde, die von einer Herabregulation von Kollagen Typ I begleitet wurde, das auf die Ränder der Mikrogewebe beschränkt war. Ein zusätzlicher die Differenzierung verstärkender Effekt wird durch das erfindungsgemäße Verfahren erreicht. Die erfindungsgemäße Fusionskultivierung verbesserte in jeder Medienbedingung die Knorpelgewebebildung im Vergleich zu einzelnen Sphäroiden. Der die Differenzierung fördernde Effekt in Fusionen ist die Zellkondensation, die das Schlüsselstadium bei der Entwicklung von Skelettgeweben ist und die selektive Regulation von für die Chondrogenese spezifischen Genen erleichtert. Fibronektin und TGF-β sind an der Kondensationsbildung beteiligt, insbesondere während der Inititierungsphase. TGF-β reguliert eine Reihe von Molekülen hinauf, die mit prächondrogenen Kondensationen assoziiert sind, einschließlich Tenascin, Fibronektin, N-CAM und N-Cadherin. Zell-Zell-Wechselwirkungen und von Gap Junctions abhängige Kommunikation sind ausschlaggebend an der chrondrogenen Differenzierung beteiligt. Adulte Gelenkknorpelchondrozyten existieren als einzelne Zellen, die in der extrazellulären Matrix eingebettet sind, und direkte intrazelluläre Kommunikation über Gap Junctions findet vorwiegend unter den abgeflachten Chondrozyten statt, die der äußeren Knorpelschicht zugewandt sind. Chondrozyten, die aus adultem Gelenkknorpel extrahiert und in einer Primärkultur gezüchtet wurden, exprimieren jedoch Connexin 43 (Cx43) und bilden funktionale Gap Junctions, die die Ausbreitung von interzellulären Kalziumwellen aufrechterhalten können. Diese Mechanismen und Komponenten, die für die Zellkondensation und -kommunikation relevant sind, erklären den überlegenen chondroinduktiven Effekt der erfindungsgemäßen Fusionskultur. Beim Starten des Fusionsprozesses wurden mehrere Schichten der Oberflächen von In-vitro-Geweben in engem Kontakt zueinander angeordnet, wodurch Zell-Zell-Wechselwirkungen und die von Gap Junctions vermittelte Kommunikation ermöglicht und dadurch damit verbundene Prozesse beeinflusst werden, wie veränderter Austausch von Ca²⁺ oder sekundärem Messenger, was zur Förderung der Knorpeldifferenzierung führt. Im Hinblick auf die mesenchymale Kondensation als einen der frühesten Schritte während der Knorpelentwicklung in vivo ahmen die einzelnen Sphäroide diesen Prozess in vitro nach. Die Kombination von mehreren einzelnen Sphäroiden mit fusionierten Mikrogeweben als zweitem Aggregationsschritt fördert die Nachahmung dieses Entwicklungsstadiums der embryonalen Knorpelbildung weiter.

S100 ist ein Marker in humanen Chondrozyten. Die Überführung von frisch isolierten postmitotischen Chondrozyten in eine Monolayerkultur führt zu einem Neustart der Proliferation, begleitet von einer Verringerung und dem Stopp der Kollagen-Typ-II-Expression in den ersten Passagen, wohingegen S100-Protein nach 4 Populationsverdopplungen oder sogar bis zu mehr als 22 PD nachgewiesen werden konnte. Parallel dazu wurde Kollagen Typ I progressiv exprimiert, was bereits in Zellen der Passage 2 begann. Die Selbstaggregation von dedifferenzierten, proliferierenden Zellen führte zu einem Proliferationsstopp und der anschließenden Expression des S100-Proteins, unabhängig von der Medienzusammensetzung und der Kulturbedingung. Die Expression von S100 war in Gegenwart von TGF-β und in allen mit der erfindungsgemäßen Fusionstechnik erzeugten Mikrogeweben noch höher. Im Gegensatz dazu waren Antikörper gegen Kollagen Typ II kaum dazu in der Lage, dieses Protein in einzelnen Sphäroiden in Basalmedium und in mit Ascorbat angereichertem Medium nachzuweisen. Um Kollagen Typ II in Mikrogeweben zu exprimieren, war eine erweiterte Stimulation des Differenzierungsprozesses erforderlich, d. h. durch Fusionsbildung und/oder biochemisch. Das beschriebene sequentielle Auftreten der Chondrozyten-Markerproteine S100 und Kollagen Typ II in differenzierenden Mikrogeweben stimmt auch mit dem Kontext überein, dass S100-Proteine Ziele des Trios der SOX-Transkriptionsfaktoren (SOX9 und dessen Koaktivatoren SOX5 und SOX6) sind und dass der Transkriptionsfaktor SOX9 wichtige Rollen in aufeinander folgenden Schritten des Chondrozyten-Differenzierungswegs spielt. Das S100-Protein wird als früher chondrospezifischer Zellmarker verwendet, der zur Qualitätskontrolle von Zellen in Kultur und sogar in In-vitro-Geweben geeignet ist. Darüber hinaus ist S100 zur Unterscheidung von Chondrozyten von anderen mesenchymalen Zellen in Bindegeweben, wie Osteoblasten oder Fibroblasten, geeignet, was ein Ausschließen einer Verunreinigung der Zellen in konstruierten knorpelartigen Gewebekonstrukten ermöglicht. Die Koexpression von Kollagen Typ II und S100 auf der Proteinebene in bestimmten Regionen von Mikrogeweben wurde in einzelnen Sphäroiden und Fusionen, die in TGF-β2-haltigem Medium kultiviert worden waren, deutlich sichtbar, was durch Immunfluoreszenz nachgewiesen wurde. Mittels Immunfluoreszenz wurde der markante, für Kollagen Typ I positive Außenrand in Kryoschnitten von einzelnen Sphäroiden und Fusionen in Gegenwart von TGF-β2 nachgewiesen. In diesen Regionen fehlte Kollagen Typ II praktisch, was eine kleine Oberflächenschicht, die fibrösem Bindegewebe ähnelt, darstellt und was Ähnlichkeiten zur Zusammensetzung von nativem Gelenkknorpel widerspiegelt, die wiederum mithilfe einer immunhistochemischen Technik nachweisbar war.

Die Kultivierung von expandierten und dedifferenzierten humanen Gelenkchondrozyten in der erfindungsgemäßen 3D-Umgebung führte zur Bildung von Mikrogeweben in Form von einzelnen Sphäroiden oder Fusionen und hat die Redifferenzierung von Zellen in jenen knorpelartigen In-vitro-Gewebekonstrukten zur Folge. Die Relevanz von S100 als Marker für die frühe Chondrozytendifferenzierung wurde durch eine zum ersten Mal beschriebene "Expressionsverschiebung" im Vergleich zu Kollagen Typ II offenbart, d. h. späte Herabregulation der S100-Expression während der Dedifferenzierung in einer Monolayerkultur, aber frühe und einfachere (nur durch Aggregation in Abwesenheit von Stimulationsfaktoren) Hinaufregulation während der Redifferenzierung in einer 3D-Kultur, bevor Kollagen Typ II überhaupt reexprimiert wurde.

Die vorliegende Erfindung stellt Daten bereit, die zeigen, dass das erfindungsgemäße Verfahren ("Fusionskulturtechnik") die chondrogene Differenzierung in vitro fördert, wodurch die Einrichtung einer selbst hergestellten extrazellulären Matrix eingeleitet wird, die sich vorwiegend aus Kollagen Typ II und Proteoglykanen zusammensetzt. In einzelnen Sphäroiden lag nur eine geringfügige Expression dieser Knorpelmarker in Basalmedium und in Gegenwart von L-Ascorbinsäure vor. Diese Einschränkung der Matrixproduktion konnte durch die Bildung von funktionalem Fusionsgewebe überwunden werden. Das erfindungsgemäße Verfahren zusammen mit geeigneten stimmulatorischen Wachstumsfaktoren induzierte synergistisch die Reexpression des Knorpelphänotyps und stellt eine Plattformtechnologie zur Erzeugung von gerüstfreien Transplantaten, Implantaten und Gewebezusammenstzungen für Menschen in vitro bereit, die klinisch beispielsweise auf die Regeneration von traumatischen Knorpeldefekten, Osteoarthritis und rheumatische Erkrankungen anwendbar sind.

Die nachfolgenden Figuren und Beispiele dienen der Erläuterung der Erfindung ohne die Erfindung jedoch auf die Figuren und Beispiele einzuschränken.

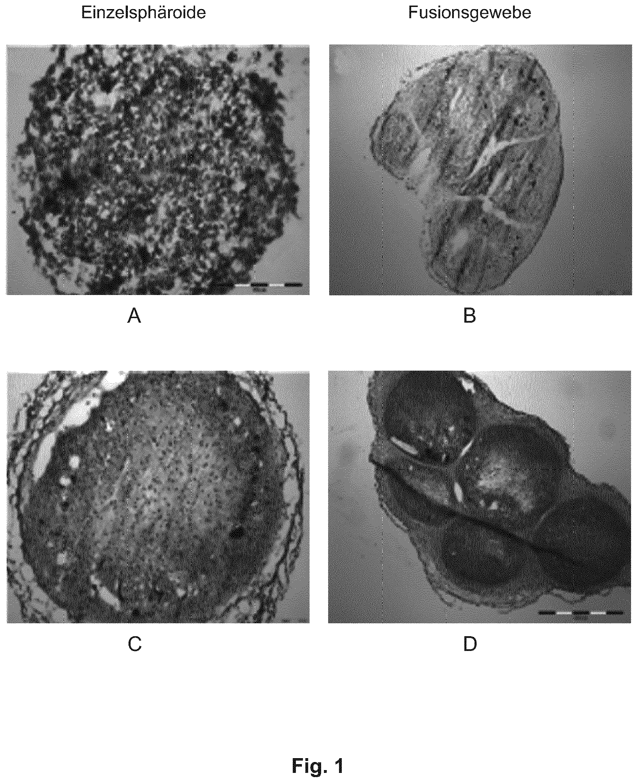

Figur 1: Darstellung der Qualitätssteigerung durch Herstellung von Fusionsgewebe aus humanen Chondrozyten anhand der Bildung einer knorpelspezifischen extrazellulären Matrix. Der Nachweis von für Knorpelgewebe charakteristischen Proteoglykanen in Einzelsphäroiden und Fusionsgeweben erfolgte mittels Safranin 0-Färbung (rot = Proteoglykane).

### A: Einzelsphäroid im Basalmedium zeigt keine

Proteoglykansynthese (keine Rotfärbung). B: Fusionskultur von 5 Einzelsphäroiden in Basalmedium induziert die Produktion von Proteoglykanen und Einbau in die extrazelluläre Matrix des Fusionsgewebes (Rotfärbung). C: Einzelsphäroid in Basalmedium angereichert mit TGF-β2 fördert Proteoglykanbildung. D: Fusionsgewebe in Gegenwart von TGF-β2 stimuliert die Synthese und Sekretion von Proteoglykanen noch deutlich stärker (intensive Rotfärbung).

Einzelsphäroide (A und C) wurden mit einer höheren Vergrößerung aufgenommen; Durchmesser ca. 1000 - 1300 µm.

Fusionsgewebe wurden mit einer geringeren Vergrößerung aufgenommen;
Maße: ca. 2000 x 3000 µm.

### Beispiele

### Beispiel 1: Zellquelle und Monolayerkultur von humanen Gelenkchondrozyten

Gelenkknorpel wurde von humanen Femurkondylen von Patienten bezogen, die einer Knieoperation unterzogen worden waren. Die Ergebnisse wurden durch Durchführung von drei unabhängigen Versuchen mit Knorpel von drei unterschiedlichen Patienten erreicht. Knorpelgewebe wurde mit einem scharfen Skalpell von den Kondylen abgeschält und Chondrozyten wurden durch mechanische Zerkleinerung des Gewebes mit einem Skalpell, gefolgt von enzymatischer Behandlung von der umgebenden Matrix isoliert. Danach wurde das gewürfelte Gewebe in Alpha-Medium und HAMs F12 (1:1) mit Kollagenase Typ II (350 E/ml) gegeben. Das geschlossene Röhrchen wurde in einen Schüttler unter Intervall-Mischen mit 300 U/min gestellt und wurde für 20 h bei 37 °C inkubiert. Die extrahierten Chondrozyten wurden bei 300 x g für 5 min zentrifugiert. Der Überstand wurde entfernt und das Pellet wurde mit 10 ml Alpha-Medium plus HAMs F12 resuspendiert, das mit 1 % L-Glutamin und 10 % Humanserum (Serumpool von freiwilligen Spendern) angereichert war und im weiteren als Basalmedium bezeichnet wird. Die Chondrozyten wurden in einer Zelldichte von 2 x 10⁴ Zellen/cm² ausplattiert. Die Zellen wurden in einer Monolayerkultur bei 37 °C und 5 % CO₂ für zwei Passagen expandiert.

### Beispiel 2: Erzeugung von knorpeligen Mikrogeweben

Um die Mikrogewebebildung zu induzieren, wurden Chondrozyten in mit Agarose beschichteten Wells von 96-Well-Platten in einer Konzentration von 3 x 10⁵ Zellen/Well in 200 µl Basalmedium pro Well gesät. Nach zwei Tagen hatten sich bereits stabile Chondrozytenaggregate gebildet, die dann unter anderen Bedingungen kultiviert wurden, um die Redifferenzierung zu fördern.

### Beispiel 3: Erzeugung von Fusionsgeweben und Bedingungen für die chondrogene Redifferenzierung

Neben den speziellen Kultivierungsbedingungen, die das Fusionieren von einzelnen Sphäroiden miteinander ermöglichen, wurden außerdem bestimmte bioaktive Substanzen verwendet, um die Redifferenzierung zu verstärken. Die Induktion von fusionierten Aggregaten wurde durch Kombinieren von fünf einzelnen Sphäroiden in einem Well einer 96-Well-Platte erzielt. Die einzelnen Sphäroide und die Fusionsgewebe wurden unter vier unterschiedlichen Bedingungen hinsichtlich des Vorliegens von bioaktiven Molekülen kultiviert. Die In-vitro-Aggregate wurden entweder in Basalmedium, das im Weiteren als BM abgekürzt wird, in mit 50 pg/ml L-Ascorbinsäure supplementiertem BM, in BM plus 5 ng/ml TGF-β2 oder in mit 50 pg/ml L-Ascorbinsäure und 5 ng/ml TGF-β2 supplementiertem BM kultiviert. Die Gesamtkultivierungszeit für alle Mikrogewebe in den erwähnten Bedingungen war sechs Wochen, wobei das Medium im Fall der einzelnen Sphäroide dreimal pro Woche und im Fall der fusionierten Mikrogewebe jeden Tag ausgetauscht wurde.

### Beispiel 4: Analyse von In-vitro-Knorpelgewebe

Nach 6 Wochen wurden die In-vitro-Gewebekonstrukte geerntet und für die weitere Analyse präpariert. Der Konstruktdurchmesser wurde mittels Bildanalyse aus dem flachen Bereich berechnet. Dieser wurde mit einem inversen Mikroskop mit Phasenkontrast CKX 41, einer Digitalkamera DP 71 und der Bildanalysesoftware Cell^{F} beurteilt. Die Gewebekonstrukte wurden in PBS gespült, in Neg-50-Gefrierschnitt-Medium eingebettet und unter Verwendung eines Kryomikrotoms geschnitten. Die 7-pm-Kryoschnitte auf Glasobjektträgern wurden an der Luft getrocknet und direkt analysiert oder bei - 20°C gelagert.

### Beispiel 5: Histologie und Immunhistochemie

Vor den Analysen wurden die Monolayer-Chondrozyten von Passage 2 und die Gewebekryoschnitte auf den Glasobjektträgern in einem Zwei-Schritt-Verfahren fixiert. Zunächst wurden sie mit 4%-igem Formaldehyd bei 4 °C für 10 min fixiert. Anschließend wurden die Objektträger in einem 1:1-Gemisch von Methanol und Aceton bei -20 °C für 10 min inkubiert. Nach diesem Fixierungsvorgang wurden die Objektträger für 3 bis 5 min in PBS gespült. Eine histologische Färbung mit Hämatoxylin und Eosin wurde zur morphologischen Analyse der Zellen und Gewebe und mit Safranin O-Fast Green zum Nachweis von Glykosaminoglykanen (GAG) durchgeführt. Die fixierten Chondrozyten und Kryoschnitte wurden immunhistochemisch für humanes Kollagen Typ I, Typ II und S100 gefärbt. Die Objektträger wurden mit PBS gespült und für 20 min bei Raumtemperatur (RT) mit Ziegennormalserum, das 1:50 in PBS/0,1%-igem BSA verdünnt worden war, inkubiert, um unspezifische Bindungen zu blockieren. Primäre Antikörper wurden wie folgt in PBS/0,1%-igem BSA verdünnt: Anti-Kollagen-Typ-I (1:1000), Anti-Kollagen-Typ-II (1:1000) und Anti-S100 (1:400). Die Zellen und die Kryoschnitte wurden mit den primären Antikörpern über Nacht bei 4 °C in einer Feuchtkammer inkubiert. Die Objektträger wurden dreimal mit PBS gewaschen und dann für 1 h im Dunkeln bei RT in einer Feuchtkammer mit Cy3-konjugiertem Ziege-Anti-Maus-Antikörper (Kollagen Typ I und II) und Ziege-Anti-Kaninchen-Antikörper (S100), die 1:600 in PBS/0,1%igem BSA mit DAPI (1 pg/ml) verdünnt worden waren, inkubiert, um die Zellkerne zu färben. Die Objektträger wurden dreimal mit PBS gewaschen, und die Zellen und Gewebeschnitte wurden anschließend in fluoreszierendem Eindeckmedium eingedeckt und mit einem Deckglas abgedeckt, um Fluoreszenzbleichung zu verhindern. Schließlich wurden die Objektträger bis zur Analyse mittels Fluoreszenzmikroskopie im Dunkeln bei 4 °C gelagert. Kryoschnitte von nativem humanem Gelenkknorpel wurden als Positivkontrolle für Kollagen Typ II und S100 und als Negativkontrolle für Kollagen Typ I verwendet. Darüber hinaus umfassten alle Versuche als Negativkontrolle den Austausch der primären Antikörper durch PBS als Kontrolle auf unspezifische Bindung des sekundären Antikörpers.

### Beispiel 6: Phasenkontrastmikroskopie für Zellkulturdokumentationen

Fotos der einzelnen Sphäroide und der Fusionen wurden in Schwarzweiß mit dem Lichtmikroskop CKX 41 aufgenommen, das mit der Kamera DP 71 ausgestattet war. Die Dokumentation erfolgte mit der Cell^{F}-Bildanalysesoftware für die Mikroskopie.

### Beispiel 7: Farbmikroskopie für histologische Proben

Die Ergebnisse der histologischen Analysen wurden unter Verwendung des Mikroskops BX 41, das mit der Kamera Color View I ausgestattet war, und der Cell^{D}-Bildanalysesoftware dokumentiert.

### Beispiel 8: Fluoreszenzmikroskopie für immunhistochemische Analysen

Die Fluoreszenz der Cy3-konjugierten Antikörper und von DAPI der immunhistochemisch gefärbten Zellen und Kryoschnitte wurde mit dem computergestützten Fluoreszenzmikroskopsystem IX81 mit einem Xenonbrenner MT20 sichtbar gemacht. Die Bilddokumentation und -auswertung wurde mit der Digitalkamera F-View II und der Cell^{R}-Bildanalysesoftware für die Mikroskopie durchgeführt.

### Beispiel 9: Zusammenfassung der Ergebnisse

### Beispiel 9.1: Analyse von humanem Gelenkknorpelgewebe

Proben von humanem hyalinem Knorpel, die von drei verschiedenen Spendern isoliert worden waren, wurden mittels Histologie und Immunhistochemie analysiert. Die Analysen einer Probe sind repräsentativ für alle Spender im Folgenden beschrieben. Die HE-Färbung zeigt die typische Struktur von humanem hyalinem Knorpel mit länglichen abgeflachten Zellen in der oberflächlichen Zone und abgerundeten Zellen, die häufig in kleinen isogenen Gruppen (Lakunen) in der mittleren Zone des Gewebes angeordnet sind. Es zeigte sich, dass die Chondrozyten (dunkelblaue Punkte stellen die Zellkerne dar) von der extrazellulären Matrix, die hellblau dargestellt ist, voneinander getrennt werden. Die SO-Fast-Green-Färbung stellt den Gehalt von Proteoglykanen im Gewebe dar. Rot gefärbte Bereiche sind Safranin-O-positiv (SO-positiv) und deuten auf Glykosaminglykane (GAG) hin. Insgesamt war die Intensität der SO-Färbung noch immer ziemlich hoch, was auf das Vorliegen von Proteoglykanen in mindestens 70 % des Gewebes hindeutet. Oberflächenregionen des Knorpelgewebeschnitts wurden jedoch von Fast Green grün gefärbt, was impliziert, dass die Proteoglykane abgebaut wurden.

Die Ergebnisse der Immunhistochemie zeigten eine überwiegende Expression von Kollagen Typ II. Die Expression dieses hyalinen Knorpel-Kennzeichens war insbesondere in den Oberflächenregionen verringert, was eine Veränderung der Matrixzusammensetzung in dieser Zone bestätigt. S100-Proteine wurden von den meisten der Zellen exprimiert, was als eine positive rote Färbung zu sehen war, die auf das Zytoplasma der Zellen beschränkt war. Wie erwartet, wurde Kollagen Typ I in nativem hyalinem Knorpel nicht exprimiert, mit Ausnahme einer sehr dünnen Schicht auf der Oberfläche. Vergleichbare histologische und immunhistochemische Ergebnisse wurden mit den Knorpelproben von zwei anderen Spendern erhalten.

### Beispiel 9.2: Dedifferenzierung der als Monolayer kultivierten humanen Chondrozyten

Während der Zellexpansion in der Monolayerkultur dedifferenzierten die Chondrozyten und erlangten eine Fibroblastenzellform. Immunhistochemische Analysen offenbarten, dass humane Chondrozyten in Passage 2 (p2) einer Monolayerkultur, d. h. nach etwa 4 Populationsverdopplungen (PD), Kollagen Typ II nur in seinem sehr geringen Ausmaß oder überhaupt nicht exprimierten. Im Gegensatz dazu wurde das Protein S100 noch immer in allen Zellen als typische punktierte Färbung exprimiert. Des Weiteren wurde das atypische Kollagen Typ I von dem Großteil der Zellen bereits nach einer solch kurzen Zeit in Kultur exprimiert.

### Beispiel 9.3: Erzeugung von In-vitro-Knorpelmikrogeweben durch spezielle 3D-Kultursysteme

Bedingungen einer dreidimensionalen Kultivierung führten zur Bildung von Mikrogeweben in Form von einzelnen Sphäroiden oder Fusionskulturen. Im Allgemeinen wurden stabile Sphäroide unabhängig von der Gegenwart von L-Ascorbinsäure und/oder TGF-β2 innerhalb von zwei Tagen gebildet und wurden in den folgenden 2 - 3 Wochen der Kultivierung kompakter, blieben jedoch in Bezug auf die Größe konstant, bis sie nach 6 Wochen geerntet wurden. Gewöhnlich lag der Durchmesser der einzelnen Sphäroide im Bereich von etwa 800 - 1400 µm. Abgesehen von Größenunterschieden zeigten die einzelnen Sphäroide keine erkennbaren morphologischen Veränderungen aufgrund von unterschiedlichen Mediensupplementen. Im Gegensatz dazu schien die Gegenwart von TGF-β2 und/oder L-Ascorbinsäure bei der Bildung der fusionierten Mikrogewebe einen Einfluss auf den Fusionsgrad zu haben, da in jeder Medienzusammensetzung, mit Ausnahme des Basalmediums, die einzelnen Sphäroide sich zu einem ziemlich kompakten Mikrogewebe verbanden, das ein kohärentes Aggregat darstellte. Obwohl die Sphäroide in dem Basalmedium an einigen Oberflächenregionen verschmolzen, bildeten die fusionierten Mikrogewebe ein eher lockeres Aggregat, in dem alle einzelnen Sphäroide wohl definiert und voneinander unterscheidbar blieben.

### Beispiel 9.4: Zell-/Matrixmorphologie und Proteoglykansynthese der In-vitro-Knorpelmikrogewebe

Die HE-Färbung von Kryoschnitten einzelner Sphäroide und der fusionierten Mikrogewebe (funktionales Fusionsgewebe) wurden analysiert. Im Fall des Basalmediums waren die Zell- und Matrixverteilungen in den einzelnen Sphäroiden und den Fusionen recht ähnlich, obwohl in den Fusionen Regionen mit erhöhter EZM-Produktion sichtbar waren, was durch einen größeren Abstand zwischen den Chondrozyten widergespiegelt wurde. Die Zugabe von L-Ascorbinsäure allein führte zu ungünstigen schwachen Gewebekonstrukten, was durch rissige Kryoschnitte widergespiegelt wurde. Die fusionierten Konstrukte waren jedoch wiederum kompakter, wie durch die gesteigerte EZM-Synthese zu erkennen war. Beide Medienzusammensetzungen, TGF-β2 allein oder in Kombination mit L-Ascorbinsäure, führten zu einer gesteigerten Matrixsynthese; insbesondere die Fusionen entwickelten jedoch eine Morphologie, die nativem Knorpel ähnlich war. Sehr bemerkenswert ist der äußere Ring mit hohem Matrixgehalt und ziemlich flachen Zellen in den einzelnen Sphäroiden und den Fusionen in Basalmedium, das mit TGF-β2 und L-Ascorbinsäure angereichert war. Die Gegenwart des Zytokins TGF-β2 in dem Kulturmedium induzierte unabhängig von der Fusionskulturtechnik eine gesteigerte Synthese von Proteoglykanen. Die Zugabe von TGF-β2 zusammen mit L-Ascorbinsäure führte zu den besten Ergebnissen in Bezug auf Safranin-O-Positivität. Im Gegensatz dazu schränkte die Wirkung von L-Ascorbinsäure allein die Proteoglykansynthese in den einzelnen In-vitro-Geweben eher ein, da nahezu keine GAG unter diesen Kulturbedingungen nachweisbar waren. Andererseits ermöglichte die Kultivierung von mehreren Sphäroiden als fusionierte Mikrogewebe die Induktion der Proteoglykansynthese unabhängig von den Differenzierungsfaktoren.

### Beispiel 9.5: Immunhistochemische Analysen der In-vitro-Knorpelmikrogewebe

Das Zytokin TGF-β2 allein oder in Kombination mit L-Ascorbinsäure förderte die Redifferenzierung von Chondrozyten in 3D-Kulturen, was zu einer erhöhten Kollagen-Typ-II-Expression führte. Insbesondere induzierte die Fusionskultivierung selbst im Vergleich zu einzelnen Gewebekonstrukten eine verstärkte Synthese von Kollagen Typ II, selbst in dem Basalmedium. Die Fusionskultivierung in Kombination mit TGF-β2-Supplementierung zeigte eine sogar noch weiter erhöhte Kollagen-Typ-II-Expression, wohingegen die Supplementierung mit L-Ascorbinsäure allein im Vergleich zum Basalmedium keine sichtbare Hinaufregulation von Kollagen Typ II induzierte.

Ähnlich der Expression von Kollagen Typ II wurde auch die S100-Expression nur aufgrund der Kultivierung von einzelnen Sphäroiden als fusionierte Mikrogewebe erhöht. Wiederum trugen die Differenzierungseffekte von TGF-β2 allein oder in Kombination mit L-Ascorbinsäure zu einer Hinaufregulation der S100-Expression bei. Es ist bemerkenswert, dass die S100-Expression korrekt mit der Lokalisierung von Kollagen Typ II in den Schnitten der einzelnen Sphäroide und der Fusionen korrelierte, insbesondere in Gegenwart von TGF-β2 allein oder plus L-Ascorbinsäure. Während Kollagen Typ II im inneren Teil der Mikrogewebe stark exprimiert wurde, war seine Expression in den äußeren Zonen sehr schwach. Ähnliche Expressionsmuster wurden für S100 beobachtet, insbesondere in Medienzusammensetzung von TGF-β2 + L-Ascorbinsäure, d. h. starke Signale in den Zentren der Gewebe, aber schwache oder sogar fehlende Signale in den äußeren Ringen.

Im Hinblick auf Kollagen Typ I als einem Marker für dedifferenzierten Knorpel wird eine verminderte Expression dieses Proteins in Gegenwart von TGF-β2 und TGF-β2 plus L-Ascorbinsäure beobachtet. In sowohl den einzelnen Sphäroiden als auch den fusionierten Mikrogeweben war die Kollagen-Typ-I-Expression auf die äußere Zone der In-vitro-Gewebe beschränkt. Darüber hinaus ist es nennenswert, dass Kollagen Typ II in Regionen, in denen Kollagen Typ I hinaufreguliert wurde, nahezu fehlte und umgekehrt.

### Beispiel 9.5: Quantitative Analyse zur Qualitätsbestimmung

### 9.5.1. Gehalt an Kollagen Typ II

Kollagen Typ II als eines der wichtigsten Bestandteile der extrazellulären Matrix im Gelenkknorpelgewebe wird in mindestens 80 bis 95% der Gewebeschnittfläche von Fusionen exprimiert. Die prozentualen Anteile ergeben sich nach computergestützter Auswertung/Berechnung der positiven Immunfluoreszenz für das Protein Kollagen Typ II bezogen auf die gesamte Gewebeschnittfläche der Fusionsgewebe.

Im Gegensatz dazu wurde in der Veröffentlichung von Anderer et al., 2002 eine Kollagen Typ II Positivität von maximal 10 % der Gewebeschnittfläche erzielt.

### 9.5.2. Gehalt an Proteoglykanen, quantitative Bestimmung

Der Proteoglykangehalt der Fusionsgewebe beträgt nach quantitativer Bestimmung durchschnittlich 300 µg pro mg Fusionsgewebe. Die Einzelmessungen ergaben Werte im Bereich von 170 - 460 µg Proteoglykane pro mg Fusionsgewebe.

Im Gegensatz dazu wurden in der Veröffentlichung von Anderer et al., 2002 keine Angaben zur Menge der Proteoglykane gemacht (keine quantitativen Daten).

## Patentansprüche

1. Verfahren zur Herstellung von funktionalem Fusionsgewebe, **dadurch gekennzeichnet, dass** Sphäroide hergestellt und Sphäroide mit einem Durchmesser von mindestens 800 µm ausgewählt werden und wobei mindestens 5 Sphäroide mit einem Durchmesser von mindestens 800 µm fusioniert werden.

2. Verfahren nach Anspruch 1, **dadurch gekennzeichnet, dass** Zellen aus Gewebe humanen oder tierischen Ursprungs isoliert werden und aus den isolierten Zellen Sphäroide hergestellt werden.

3. Verfahren nach einem der vorgehenden Ansprüche, **dadurch gekennzeichnet, dass** die isolierten Zellen vermehrt und aus den vermehrten Zellen Sphäroide hergestellt werden.

4. Verfahren nach einem der vorgehenden Ansprüche, **dadurch gekennzeichnet, dass** Sphäroide durch Kultivierung von mindestens 3 x 10⁵ Zellen pro Well einer 96-Well-Platte hergestellt werden.

5. Verfahren nach einem der vorgehenden Ansprüche, **dadurch gekennzeichnet, dass** Sphäroide durch Kultivierung der Zellen für 1 bis 3 Tage hergestellt werden.

6. Verfahren nach einem der vorgehenden Ansprüche, **dadurch gekennzeichnet, dass** die Fusion durch gemeinsame Kultivierung von 5 oder mehr Sphäroiden für einen Zeitraum von 3 bis 7 Wochen erfolgt.

7. Verfahren nach einem der vorgehenden Ansprüche, **dadurch gekennzeichnet, dass** die Sphäroide für die Fusion auf eine konkave Oberfläche aufgebracht werden.

8. Verfahren nach einem der vorgehenden Ansprüche, **dadurch gekennzeichnet, dass** die Sphäroide in Gegenwart von einem oder mehreren Differenzierungsinduktor(en) fusioniert werden und wobei der/die Differenzierungsinduktor(en) ausgewählt werden aus mechanischen, chemischen oder biochemischen Differenzierungsinduktoren.

9. Verfahren nach einem der vorgehenden Ansprüche, **dadurch gekennzeichnet, dass** die Zellen aus Gewebe endodermalen, ektodermalen oder mesodermalen Ursprungs isoliert werden, beispielsweise aus muskoskelletalem Gewebe, Skelettgewebe, Knorpel, Knochen, Meniskus, Epithelgewebe, Bindegewebe, Stützgewebe, Muskelgewebe, glatter Muskulatur, Herzmuskulatur, Nervengewebe, Funktionsgewebe (Parenchym), Zwischengewebe (Interstitium), Organgewebe, beispielsweise von Leber, Niere, Nebennierenrinde, Magen, Bauchspeicheldrüse, Herz, Lunge, Haut, Augenhornhaut, subkutanem Gewebe, Verdauungstrakt, Knochenmark, Gehirn, Schilddrüse, Milz, Gelenk, Sehne.

10. Verfahren nach einem der vorgehenden Ansprüche, **dadurch gekennzeichnet, dass** die Zellen Chrondrozyten umfassen.

11. Funktionales Fusionsgewebe, erhältlich durch ein Verfahren nach einem der Ansprüche 1 bis 10, wobei mindestens 5 Sphäroide mit einem Durchmesser von mindestens 800 µm fusioniert sind.

12. Arzneimittel, pharmazeutische Zubereitung, Transplantat oder Implantat umfassend ein funktionales Fusionsgewebe nach Anspruch 11 und gegebenenfalls weitere Zusatz- und Hilfsstoffe.

13. Funktionales Fusionsgewebe, Arzneimittel, Transplantat oder Implantat nach einem der Ansprüche 11 bis 12 zur Verwendung in der Behandlung von rheumatischen Erkrankungen, Knorpeldefekten, Knochendefekten, insbesondere traumatischen Knorpeldefekten und/oder Knochendefekten, Läsionen, insbesondere traumatischen Läsionen, bei Knorpeldegenerationen, Knochendegeneration, Osteoarthritis, zur therapeutische Knorpelregeneration und / oder Knochenregeneration in vitro oder in vivo.

14. Nahrungsmittel umfassend ein funktionales Fusionsgewebe nach Anspruch 11.

15. Testsystem oder Test-Kit, umfassend
a) funktionales Fusionsgewebe, ein Arzneimittel, ein Transplantat oder Implantat nach einem der Ansprüche 11 bis 13,
b) gegebenenfalls weitere Hilfs- und Zusatzstoffe und
c) Mittel zum Nachweis.

16. Verfahren zum Testen von zu untersuchenden Substanzen **dadurch gekennzeichnet, dass**
a) funktionales Fusionsgewebe, ein Arzneimittel, ein Transplantat oder Implantat nach einem der Ansprüche 11 bis 13,
b) mit einer oder mehreren zu untersuchenden Substanze in Kontakt gebracht wird,
c) der Effekt von der/den zu untersuchenden Substanz(en) auf das funktionale Fusionsgewebe, das Arzneimittel, das Transplantat oder Implantat nachgewiesen wird.

## Claims

1. A method for producing functional fusion tissue, **characterised in that** spheroids are produced and spheroids having a diameter of at least 800 µm are selected, and wherein at least 5 spheroids having a diameter of at least 800 µm are fused.

2. The method according to claim 1, **characterised in that** cells from tissue of human or animal origin are isolated and spheroids are produced from the isolated cells.

3. The method according to either one of the preceding claims, **characterised in that** the isolated cells are propagated and spheroids are produced from the propagated cells.

4. The method according to any one of the preceding claims, **characterised in that** spheroids are produced by cultivating at least 3 x 10⁵ cells per well of a 96-well plate.

5. The method according to any one of the preceding claims, **characterised in that** spheroids are produced by cultivating the cells for 1 to 3 days.

6. The method according to any one of the preceding claims, **characterised in that** the fusion is performed by joint cultivation of 5 or more spheroids for a period of from 3 to 7 weeks.

7. The method according to any one of the preceding claims, **characterised in that** the spheroids are applied to a concave surface for the fusion.

8. The method according to any one of the preceding claims, **characterised in that** the spheroids are fused in the presence of one or more differentiation inducer(s), and wherein the differentiation inducer(s) is/are selected from mechanical, chemical or biochemical differentiation inducers.

9. The method according to any one of the preceding claims, **characterised in that** the cells are isolated from tissue of endodermal, ectodermal or mesodermal origin, for example from musculoskeletal tissue, skeletal tissue, cartilage, bone, meniscus, epithelial tissue, connective tissue, supporting tissue, muscular tissue, smooth muscle, heart muscle, nerve tissue, functional tissue (parenchyma), intermediate tissue (interstitium), or organ tissue, for example from liver, kidney, adrenal cortex, stomach, pancreas, heart, lung, skin, cornea, subcutaneous tissue, intestinal tract, bone marrow, brain, thyroid, spleen, joint, or tendon.

10. The method according to any one of the preceding claims, **characterised in that** the cells comprise chondrocytes.

11. A functional fusion tissue obtainable by a method according to any one of claims 1 to 10, wherein at least 5 spheroids having a diameter of at least 800 µm are fused.

12. A medicinal drug, pharmaceutical preparation, transplant or implant comprising a functional fusion tissue according to claim 11 and optionally further additives and auxiliaries.

13. A functional tissue, medicinal drug, transplant or implant according to any one of claims 11 to 12 for use in the treatment of rheumatic diseases, cartilage defects, bone defects, in particular traumatic cartilage defects and/or bone defects, lesions, in particular traumatic lesions, in cartilage degeneration, bone degeneration, osteoarthritis, for therapeutic cartilage regeneration and/or bone regeneration *in vitro* or *in vivo.*

14. A food comprising a functional fusion tissue according to claim 11.

15. A test system or test kit, comprising
a) functional fusion tissue, a medicinal drug, a transplant or implant according to any one of claims 11 to 13,
b) optionally further auxiliaries and additives, and
c) detection means.

16. A method for testing substances that are to be examined, **characterised in that**
a) functional fusion tissue, a medicinal drug, a transplant or an implant according to any one of claims 11 to 13,
b) is brought into contact with one or more substances to be examined,
c) the effect of the substance(s) to be examined on the functional fusion tissue, the medicinal drug, the transplant or the implant is detected.

## Revendications

1. Procédé de fabrication d'un tissu de fusion fonctionnel, **caractérisé en ce que** des sphéroïdes sont fabriqués et des sphéroïdes avec un diamètre d'au moins 800 µm sont sélectionnés et où au moins 5 sphéroïdes avec un diamètre d'au moins 800 µm sont fusionnés.

2. Procédé selon la revendication 1, **caractérisé en ce que** des cellules provenant de tissus humains ou d'origine animale sont isolées et des sphéroïdes sont fabriqués à partir des cellules isolées.

3. Procédé selon l'une des revendications précédentes, **caractérisé en ce que** les cellules isolées sont multipliées et des sphéroïdes sont fabriqués à partir des cellules multipliées.

4. Procédé selon l'une des revendications précédentes, **caractérisé en ce que** des sphéroïdes sont fabriqués par la culture d'au moins 3 x 105 cellules par puits d'une plaque de 96 puits.

5. Procédé selon l'une des revendications précédentes, **caractérisé en ce que** des sphéroïdes sont fabriqués par la culture des cellules pendant 1 à 3 jours.

6. Procédé selon l'une des revendications précédentes, **caractérisé en ce que** la fusion a lieu par une culture commune de 5 ou plus de sphéroïdes pendant une durée de 3 à 7 semaines.

7. Procédé selon l'une des revendications précédentes, **caractérisé en ce que** les sphéroïdes sont rapportés sur une surface concave pour la fusion.

8. Procédé selon l'une des revendications précédentes, **caractérisé en ce que** les sphéroïdes sont fusionnés en présence d'un ou de plusieurs inducteur(s) de différentiation et où l'inducteur/les inducteurs de différentiation sont choisis parmi des inducteurs de différentiation mécaniques, chimiques ou biochimiques.

9. Procédé selon l'une des revendications précédentes, **caractérisé en ce que** les cellules sont isolées à partir de tissu d'origine endodermique, ectodermique ou mésodermique, par exemple, à partir de tissus musculo-squelettiques, de tissu squelettique, de cartilage, d'os, de ménisque, de tissu épithélial, de tissu conjonctif, de tissu de support, de tissu musculaire, de muscles lisses, de muscle cardiaque, de tissu nerveux, de tissu fonctionnel (parenchyme), de tissu interstitiel (interstitium), de tissu d'organe, par exemple, provenant du foie, du rein, du cortex surrénal, de l'estomac, du pancréas, du coeur, du poumon, de la peau, de la cornée, de tissu sous cutané, d'extrait digestif, de moelle osseuse, de cerveau, de thyroïde, de rate, d'articulation, de tendon.

10. Procédé selon l'une des revendications précédentes, **caractérisé en ce que** les cellules comprennent des chondrocytes.

11. Tissu de fusion fonctionnel pouvant être obtenu par un procédé selon l'une des revendications 1 à 10, où au moins 5 sphéroïdes avec un diamètre d'au moins 800 µm sont fusionnés.

12. Produit pharmaceutique, préparation pharmaceutique, transplant ou implant comprenant un tissu de fusion fonctionnel selon la revendication 11 et éventuellement d'autres substances complémentaires et auxiliaires.

13. Tissu de fusion fonctionnel, produit pharmaceutique, transplant ou implant selon l'une des revendications 11 à 12 destiné à l'emploi dans le traitement de maladies rhumatismales, de défauts de cartilage, de défauts osseux, notamment de défauts de cartilage et/ou de défauts osseux traumatiques, de lésions, notamment de lésions traumatiques, lors de dégénération du cartilage, de dégénération osseuse, d'ostéoarthrite, pour la régénération thérapeutique de cartilage et/ou la régénération osseuse in vitro ou in vivo.

14. Produit alimentaire comprenant un tissu de fusion fonctionnel selon la revendication 11.

15. Système de test ou ensemble de test comprenant
a) du tissu de fusion fonctionnel, un produit pharmaceutique, un transplant ou un implant selon l'une des revendications 11 à 13,
b) éventuellement, d'autres substances auxiliaires et complémentaires, et
c) un moyen pour la détection.

16. Procédé de test de substances à rechercher **caractérisé en ce que**
a) du tissu de fusion fonctionnel, un produit pharmaceutique, un transplant ou un implant selon l'une des revendications 11 à 13,
b) est mis en contact avec une ou plusieurs des substances à rechercher,
c) l'effet de la/des substance(s) à rechercher est à attribuer au tissu de fusion fonctionnel, au produit pharmaceutique, au transplant ou à l'implant.
